# EUROPEAN PATENT SPECIFICATION

(11) **EP 2 918 685 B1**
(45) Date of publication and mention of the grant of the patent: **02.01.2019**
(21) Application number: 15158378.8
(22) Date of filing: 10.03.2015
(51) Int. Cl.: C12Q 1/6888

(54) **NUCLEOTIDE AND AMMINOACID SEQUENCES OF PHYTOPLASMAS CAUSING FLAVESCENCE DOREE AND OF PHYLOGENETICALLY SIMILAR PHYTOPLASMAS, AND USE OF SUCH SEQUENCES**
NUKLEOTID- UND AMMINOACIDSEQUENZEN VON PHYTOPLASMEN, DIE GOLDGELBE VERGILBUNG VERURSACHEN, UND VON PHYLOGENETISCH ÄHNLICHEN PHYTOPLASMEN UND VERWENDUNG VON DIESEN SEQUENZEN
SÉQUENCES DE NUCLÉOTIDES ET D'ACIDES AMINÉS DE PHYTOPLASMAS PROVOQUANT LA FALVESCENCE DORÉE ET DE PHYTOPLASMAS SIMILAIRES D'UN POINT DE VUE PHYLOGÉNÉTIQUE ET UTILISATION DE CES SÉQUENCES

(30) Priority: 12.03.2014 IT TO20140195
(43) Date of publication of application: 16.09.2015
(73) Proprietor: Consiglio per la ricerca in agricoltura e l´analisi dell´economia agraria (CRA), 00184 Roma (IT)
(72) Inventor: Filippin, Luisa, 31015 Conegliano (TV) (IT); Angelini, Elisa, 31015 Conegliano (TV) (IT)
(74) Representative: Robba, Pierpaolo

(56) References cited:
- WO-A1-2012/017271
- Filippin Luisa: "Scientific Report of Luisa Filippin on the STSM (ref. code: COST-STSM-FA0807-05285)", , 2010, pages 1-4, XP055133519, Retrieved from the Internet: URL:http://www.costphytoplasma.ipwgnet.org /PDF files/STSM/Luisa Filippin Report for COST Website.pdf [retrieved on 2014-08-06]
- DA ROLD G ET AL: "The imp gene in Flavescence dorée and related phytoplasmas from grapevine, clematis and alder", 18TH INTERNATIONAL CONGRESS OF INTERNATIONAL ORGANISATION OF MYCOPLASMOLOGY (IOM CONGRESS 2010), SIENA, ITALY, JULY 10-16 2010,, 10 July 2010 (2010-07-10), XP009179542,
- G. Da Rold, L. Filippin, M. Borgo and E. Angelini: "Characterization of the imp gene in flavescence dorée andrelated phytoplasmas", , 2010, page 100, XP002740629, Retrieved from the Internet: URL:http://www.costphytoplasma.ipwgnet.org /PDF%20files/WG%20BookwithiISBN.pdf [retrieved on 2015]
- Luisa Filippin ET AL: "Nucleotide sequencing of imp gene in phytoplasmas associated to 'flavescence dorée' from Ailanthus altissima", Bulletin of Insectology, 1 January 2011 (2011-01-01), pages 49-50, XP055133521, Retrieved from the Internet: URL:http://www.bulletinofinsectology.org/p dfarticles/vol64-2011-S049-S050filippin.pd f [retrieved on 2014-08-06]
- "Grapevine flavescence dorée phytoplasma", PO BULLETIN: A JOURNAL OF REGULATORY PLANT PROTECTION,, vol. 37, no. 3, 1 December 2007 (2007-12-01), pages 536-542, XP002609127, BLACKWELL SCIENTIFIC PUBLICATIONS, OXFORD, GB ISSN: 0250-8052, DOI: 10.1111/J.1365-2338.2007.01161.X [retrieved on 2007-12-07]
- DATABASE EMBL [Online] 22 April 2014 (2014-04-22), Rashidi,M., Galetto,L. and Marzachi,C.: "Candidatus Phytoplasma vitis DnaD (dnaD) gene, partial cds; Imp (imp) gene, complete cds; and PyrG (pyrG) gene, partial cds.", XP002730362, retrieved from EBI Database accession no. KJ402359.1

### SEQUENCE LISTING

<110> CONSIGLIO PER LA RICERCA E LA SPERIMENTAZIONE IN AGRICOLTURA - CRA
<120> SEQUENZE NUCLEOTIDICHE E AMMINOACIDICHE DI FITOPLASMI RESPONSABILI
   DELLA FLAVESCENZA DORATA E FITOPLASMI FILOGENETICAMENTE SIMILI, E LORO USO
<130> P4157IT00
<140> ITT02014A000195
   <141> 2014-03-12
<160> 86
<170> BiSSAP 1.3
<210> 1
   <211> 827
   <212> DNA
   <213> 16SrV (Elm yellows group)
<400> 1
   aaaataattt aaaaatagaa aataatgata aagcagataa aattcttcat aaaattttta      60
   ataaaattaa ataatttaaa atataacata tataataaaa tatgttataa tatttgtatt     120
   gaatctaaga aaagatttaa taaaatgtat ttacatttta aatactgtat attaaatacc     180
   gtataaatta taatttaatg agaggaaaga agaaaatgca acaaaaagaa agttttttaa     240
   aaactaaaaa aggcaaaata gttgttggtt cagttggttc agttattggt gctttagtat     300
   tatatttaat aattgcttat gttatgaaat ggattccatt tagggaaaaa attactccgg     360
   aagatgcaga aagtttaaaa acaaaaatag aatcattagt agcagatact gaagcaaaaa     420
   caactgtagc agacaatgac aaaatatatg acaaagtaaa aaaagcaatt gaagcttttg     480
   aatcaaaagt aacagcagtt aatgacaaaa gagctacaga aaaaaaaatt gatactactg     540
   aaatagcaaa tttaaaaaca gcagttgaag gtttaaaaaa tcaagatact caagaaaatt     600
   ttaaagcagc agcaaaaaat ttcaaagtaa aagttgctaa ttcaataaca aatttaaaaa     660
   cagtaattac agaaaaaaac aaataataat ttaaacaata atattaatta atattattta     720
   aaaataaaaa aaacattcaa aatattaaat tttgaatgtt tttttatttt atattcgaat     780
   aaaattgtaa agatgctctt ataaagtctt taaataaagg gtgtggt                   827
<210> 2
   <211> 771
   <212> DNA
   <213> 16SrV (Elm yellows group)
<400> 2
   aaataatgat aaagcagata aaattcttca taaaattttt aataaaatta aataatttaa      60
   aatataacat atataataaa atatgttata atatttgtat tgaatctaag aaaagattta     120
   ataaaatgta tttacatttt aaatactgta tattaaatac cgtataaatt ataatttaat     180
   gagaggaaag aagaaaatgc aacaaaaaga aagtttttta aaaactaaaa aaggcaaaat     240
   agttgttggt tcagttggtt cagttattgg tgctttagta ttatatttaa taattgctta     300
   tgttatgaaa tggattccat ttagggaaaa aattactccg gaagatgcag aaagtttaaa     360
   aacaaaaata gaatcattag tagcagatac tgaagcaaaa acaactgtag cagacaatga     420
   caaaatatat gacaaagtaa aaaaagcaat tgaagctttt gaatcaaaag taacagcagt     480
   taatgacaaa agagctacag aaaaaaaaat tgatactact gaaatagcaa atttaaaaac     540
   agcagttgaa ggtttaaaaa atcaagatac tcaagaaaat tttaaagcag cagcaaaaaa     600
   tttcaaagta aaagttgcta attcaataac aaatttaaaa acagtaatta cagaaaaaaa     660
   caaataataa tttaaacaat aatattaatt aatattattt aaaaataaaa aaaacattca     720
   aaatattaaa ttttgaatgt ttttttattt tatattcgaa taaaattgta a              771
<210> 3
   <211> 885
   <212> DNA
   <213> 16SrV (Elm yellows group)
<400> 3
   aatttaaaaa tagaaaataa cgataaagca gataaaattc ttcataaaat ttttaataaa      60
   attaaataat ttaaaatata acaaatataa taaaatatgt tataatattt gtattgaatc     120
   taagaaaaga tttaataaaa tgtatttaca ttttaaatac tgtatattag ataccgtata     180
   aattataatt ttaatgagag gaaagaagaa aatgcaacaa aaagaaagtt ttttaaaaac     240
   taaaaaaggc aaaatagttg ttggttcagt tggttcagtt gttggtgttt tagtattatt     300
   tttaataatt gcttattttg cgtcattttg gccatttaat ccaacaatta ctgagaaaga     360
   tttaacaaca tttaaaacag atgtagaaaa attagtagaa gatggaaaag caacagatgt     420
   taaagcagac aataacaaaa tagtagaagc agtagaaaac aaaattaacg ctttgaaaac     480
   acaagtggaa caagttaata aaaaaagagc tgacaacaaa aaaattaaaa cagaaaaaat     540
   agacacttta aaaacagaag ctgtcaaatt aaaagacaaa gaaacaaaag tagcatttaa     600
   agaagaagct acgaaattca aaacaaaagt tgatacattc gtaactgaat tcaaagaaga     660
   aattaaagaa aaataattta aacaataata ttaattaata ttatttaaaa ataaaaaaaa     720
   cattcaaaat ttaatatttt gaatgttttt tttattttat atttgaataa aattgtaaag     780
   atgctcttat aaaatcttta aataaaggat gtggtttaaa aggtcttgat aaaaattcag     840
   aatgaaaatt gaacctccta taaaccaaat atgttttttt aaatt                      885
<210> 4
   <211> 1281
   <212> DNA
   <213> 16SrV (Elm yellows group)
<220>
   <221> allele
   <222> 1269..1279
<400> 4
   ccttacaaga tgtaaaaaga tctataaata tagcttacac aaatcaaaaa aaatcaattt 60
   attatataga gagaatttta ggccacgaaa ataatttaaa aatagaaaat aacgataaag 120
   cagataaaat tcttcataaa atttttaata aaattaaata atttaaaata taacaaatat 180
   aataaaatat gttataatat ttgtattgaa tctaagaaaa gatttaataa aatgtattta 240
   cattttaaat actgtatatt aaataccgta taaattataa ttttaatgag aggaaagaag 300
   aaaatgcaac aaaaagaaag ttttttaaaa actaaaaaag gcaaaatagt tgttggttca 360
   gttggttcag ttgttggtgt tttagtatta tttttaataa ttgcttattt tgcgtcattt 420
   tggccattta atccaacaat tactgagaaa gatttaacaa catttaaaac agatgtagaa 480
   aaattagtag aagataaaaa agcaacagat gttgaagcag acaatgacaa aatagtatta 540
   gatgtagaaa aaaaaattaa cgctttaaaa acacaagtag aagaagttaa taaaaaaaga 600
   gatgacgaca aaaaaattaa aacagaaaaa atagacaatt taaaaacaga agctggcaaa 660
   ttaaaaggca aaaaaacaaa agacacattt aaagatgaag ctaagaaatt caaagcaagc 720
   gttgatacat tcgtaactga atacaaaaca gacattaaaa aataataatt taaaaaacga 780
   tattaattaa tattatttaa aaataaaaaa aaacattcaa aaatttaata ttttgaatgt 840
   tttttttatt ttatattcga ataaaattgt aaagatgctc ttataaagtc tttaaataaa 900
   gggtgtggtt taaaaggtct tgataaaaat tcagaatgaa attgaactcc tataaaccaa 960
   atatgttttt ttaattctat aacttcaggt aattgttctt cattattaat tcctgaaata 1020
   ataaaattat catctttatc aaaaagtgaa acatattcca tattcatctc ataacgatgt 1080
   ctatgtcttt catgaataat ttctttttga aaaatttcat aactcttagt atttttcttt 1140
   aaacaacaag agtataatcc taatcttaaa gttcctccta ataaaggatc ttttattttt 1200
   tgagttatta aaggaagtgg cgtattaata tcaatttctg tagaattagc tttgtctaat 1260
   tcaaaacang cnagcaatnc a 1281
<210> 5
   <211> 727
   <212> DNA
   <213> 16SrV (Elm yellows group)
<400> 5
   aatataatta aaatatgkta taatatttgt attgaatcta agaaaagatt taataaaatg 60
   tatttacatt ttaaatactg tatattaaat accgtataaa ttataatttt aatgagagga 120
   aagaagaaaa tgcaacaaaa agaaagtttt ttaaaaacta aaaaaggcaa aatagttgtt 180
   ggttcagttg gttcagttgt tggtgtttta gtattatatt taataattgc ttattttgcg 240
   tcactttatc catttaatac aacaattact gagaaagatt taacaaaatt taaaacagat     300
   gtagaagaat tagtagcaga tggaaaagaa acagatgcta aagtagacaa taacaaaata     360
   gtagaagcag tagaaaaaaa aattaacgct ttgaaagaac aagtggaaaa agttaataaa     420
   aaaagagctg acaacaaaaa aattaaaaca gaaaaagtag acactttaaa aacagaagct     480
   ggcaaattaa aagacaaaga aacaaaaata gcatttaaag aagaagctac gaaattcaaa     540
   acaaaagtta atacattcgt aactgaattc aaagaagaaa ttaaagaaaa ataatttaaa     600
   aaataatatt aattaatatt atttaaaaat aaaaaaaaca ttcaaaattt aatattttga     660
   atgttttttt tattttatat tcgaataaaa ttgtaaagat gctcttataa agtctttaaa     720
   taaagga                                                               727
<210> 6
   <211> 768
   <212> DNA
   <213> 16SrV (Elm yellows group)
<400> 6
   aatttaaaat agaaaataac gataaagcag ataaaattct tcataaaatt tttaataaaa      60
   ttaaataatt taaaatataa caaatataat aaaatatgtt ataatattga tagttgaatc     120
   taagaaaaga tttaataaaa tgtatttaca ttttaaatac tgtatattaa ataccgtata     180
   aattataatt taatgagagg aaagaagaaa atgcaacaaa aagaaagttt tttaaaaact     240
   aaaaaaggta aaatagtttt tggttcagtt gttggtgttt tagcattata tttactaatt     300
   gcttattttg cgtcattttg gccatttaat acaacaatta ctgagaaaga tttaacaaca     360
   tttaaaacag atgtagaaaa attagtagca gatgaaaaag caacagatgt tgaagcagac     420
   aatgacaaaa tagtattaga tgtagaaaaa aaaattaacg ctttaaaaac acaagtagaa     480
   gaagttaata aaaaaagagc tgacgacaaa aaaattaaac cagaaaaaat agacaattta     540
   aaaatagaag ctgacaaatt aaaaggcaaa aaaacaaaag acacatttaa agatgaagct     600
   aagaaattca aagcaagcgt tgataaattc gtaactgaat acaaaacaga cattaaataa     660
   taataattta aaaaatgata ttaattaata ttatttaaaa ataaaaaaaa cattcaaaaa     720
   tttaatattt tgaatgtttt ttttatttta tattcgaata aaattgta                  768
<210> 7
   <211> 787
   <212> DNA
   <213> 16SrV (Elm yellows group)
<400> 7
   aaataattta aaaatagaaa ataaccgata aagcagataa aattcttcat aaaattttta 60
   ataaaattaa ataatttaaa atataacaaa tataataaaa tatgttataa tattgatagt 120
   tgaatctaag aaaagattta ataaaatgta tttacatttt aaatactgta tattaaatac 180
   cgtataaatt ataattttaa tgagaggaaa gaagaaaatg caacaaaaag aaagtttttt 240
   aaaaactaaa aaaggcaaaa tagttgttgg ttcagttggt tcagttgttg gtgttttagt 300
   attatattta ataattgctt atattatgaa atgggtacca tttggtatat caattaataa 360
   tacagattta acaacattta aggcagatgt agaaaaatta gtagcagatg aaaaagcaac 420
   agatgttaaa gcagataata acaaaatagt agaagcagta gaaaacaaaa ttaacgcttt 480
   gaaaacacaa gtggaacaag ttaatgaaaa aagagctaac aaaaaaatta aaacagaaaa 540
   aatagacact ttaaaaacag aagctggcaa attaaaagac aaagaaacaa aaacagcatt 600
   taaagaagaa gctacgaaat tcaaaacaag cgttgataaa ttcgtaactg aattcaaaga 660
   agaaattaaa gaataataat ttaaacaata atattaatta atattattta aaaataaaaa 720
   aaacattcaa aatttaatat tttgaatgtt ttttttattt tatatttgaa taaaattgta 780
   aagatgc 787
<210> 8
   <211> 778
   <212> DNA
   <213> 16SrV (Elm yellows group)
<400> 8
   cgataaagca gataaaattc ttcataaaat ttttaataaa attaaataat ttaaaatata      60
   acaaatataa taaaatatgt tataatattt gtattgaatc taagaaaaga tttaataaat     120
   gtatttacat tttaaatact gtatattaaa taccgtataa attataattt taatgagagg     180
   aaagaagaaa atgcaacaaa aagaaagttt tttaaaaact aaaaaaggca aaatagttgt     240
   tggttcagtt ggttcagttg ttggtgtttt agtattatat ttaatagttg cttatattat     300
   atcaatgtgg ccattcgctg caaaaattac tgagaaagat ttagcaacat ttaaaacaga     360
   tgtagaaaaa ttagtagcag atgaaaaagc aggttctaaa acagaaaatg acacaacagc     420
   aaaaaaagta gaagaaagaa tcgaagaagt gaaaaagcta gtagacaaag ttaatgaaaa     480
   aagagcttca gacaaaaaaa ttgacacaaa aaaaatagaa gacttaaaaa cagaagctgg     540
   caaattaaaa gacaaagaaa aagaagcatt taaaacagta gctgcagaat tcaaaacaaa     600
   agtcggtagt ttcgtaacta gtttcaaaga agacattaaa gaaaaataat ttaaaaaatg     660
   atattaatta atattattta aaaataaaaa aaacattcaa aatttaatat tttgaatgtt     720
   ttttttattt tatattcgaa taaaattgca aagatgctct tataaagtct ttaaataa        778
<210> 9
   <211> 863
   <212> DNA
   <213> 16SrV (Elm yellows group)
<400> 9
   ataggaaata acgataaagc agataaaatt cttcataaaa tttttaataa aattaaataa 60
   tttaaaatat aacaaatata ataaaatatg ttataatgat gttgaatcta agaaaagatt 120
   taataaaatg tatctacatt ttaaataatg tagaaattat taattttaat gagaggaaag 180
   aaaaaaatgc aacaaaaaga aagtttttta aaaactaaaa aaggcaaaat agttgttggt 240
   tcagttgttg gttcaattgt tggtttatta gcaattttaa cactttgtta tattatgtca 300
   tggttcccat tcagtccatc aattaatgaa acagatgtaa acaaatttgg agataaagtt 360
   aaagcattaa tagtagatgg taaagtaaaa gattctaaag atgacaataa caaaatattt 420
   gaagatgtga caaaagaaat tgatactttg aaaaaaaaag tagaagcagt taataacaaa 480
   agaaatacag acaaaaaaat taaaacagaa gaaatagaca aattaaaaac aaaagctgaa 540
   gaattaaaag acaaagaaaa aactgcattt aaaacagcag ctgttaaatt caaagcagaa 600
   gtcgaaaatt tattagatag tttaaaatca gaaattaata gtaaaaaata atttcaacaa 660
   taatattaat taatattatt taaaaataaa aaaaacattc aaaatattca attttgaatg 720
   ttttttttat tttatatttg aataaaattg taaagatgct cttataaagt ctttaaataa 780
   aggatgcggt ttaaaaggtc ttgataaaaa ttcagaatga aactgaactc ctataaactc 840
   caaattatgt ttttttaatt cta 863
<210> 10
   <211> 583
   <212> DNA
   <213> 16SrV (Elm yellows group)
<400> 10
   tgagaggaaa gaagaaaatg caacaaaaag aaagtttttt aaaaactaaa aaaggcaaaa 60
   tagtttttgg ttcagttggt tcagttgttg gtgttttagt attatattta ataattgctt 120
   attttgtatc aatttggcca tttagtccat caattactaa taaagattta acaacattta 180
   aaacagatgt agaaaaatta gtaacagaca aagaaacaga tgttaaatta gaaaatgaca 240
   aaatagcaac agaagtagaa aaaaaaattg acgatttgaa aacacaagtg ataaaggtta 300
   ataaaaaaag agctgacaac aaacaaatta aaacagaaaa aatagacaat ttaaaaacag 360
   aagctggcaa attaaaaggc aaagaaacaa aagcagcatt taaagatgaa gctacaaaat 420
   tcaaaacaaa cgttgataca ttcataaata gtttcaaaga agacattaaa gaataattta 480
   aacaataaaa tattaattaa tattatttaa aaataaaaaa aacattcaaa atttaatatt 540
   ttgaatgttt tttttatttt atatttaaat aaaattttaa aga 583
<210> 11
   <211> 895
   <212> DNA
   <213> 16SrV (Elm yellows group)
<400> 11
   aaaataatta aaaatagaaa ataacgataa agcagataaa attcttcata aaatttttaa      60
   taaaattaaa taatttaaaa tataacaaat ataataaaat atgttataat atttgtattg     120
   aatctaagaa aagatttaat aaaatgtatt tacattttaa atactgtata ttaaataccg     180
   tataaattat aattttaatg agaggaaaaa agaaaatgca acaaaaagaa agttttttaa     240
   aaactaaaaa aggcaaaata gttgttggtt cagttggttc agttgttggt gttttagtat     300
   tatttttaat aattgcttat tttgcgtcag tttggccatt taatccaaca attactgaga     360
   aagatttaac aacatttaaa acagatgtag aaaaattagt agaagatgga aaagcaacag     420
   atgttaaagc agacaataac aaaatagtag aagcagtaga aaacaaaatt aacgctttga     480
   aaacacaagt ggaacaagtt aataaaaaaa gagctgacaa caaaaaaatt aaaacagaaa     540
   aaatagacac tttaaaaaca gaagctgtca aattaaaaga caaagaaaca aaagtagcat     600
   ttaaagaaga agctacgaaa ttcaaaacaa aagttgatac attcgtaact gaattcaaag     660
   aagaaattaa agaaaaataa tttaaacaat aatattaatt aatattattt aaaaataaaa     720
   aaaacattca aaatttaata ttttgaatgt tttttttatt ttatatttga ataaaattgt     780
   aaagatgctc ttataaaatc tttaaataaa ggatgtggtt taaaaggtct tgataaaaat     840
   tcagaatgaa attgaactcc tataaaccaa atatgttttt ttaattctat aactt          895
<210> 12
   <211> 651
   <212> DNA
   <213> 16SrV (Elm yellows group)
<400> 12
   tttaatgaga ggaaagaaga aaatgcaaca aaaagaaagt tttttaaaaa ctaaaaaagg      60
   caaaatagtt gttggttcag ttggttcagt tgttggtgtt ttagtattat ttttaataat     120
   tgcttatttt gcgtcaattt ggccatttag tacaaaaatt actaataaag atttagaaaa     180
   atttaaaaca gatgtagaaa aattagtaga agatgaaaaa gcaaaagatt cgaaaacaga     240
   aaataacaaa atagtagaaa cagtagaaaa caaaattaac gctttgaaaa cacaagtgac     300
   aacagttaat gaaaaaagag ctgacaacaa aaaaattaaa acagaaaaaa tagacacttt     360
   aaaaacagaa gctgccaaat taaaagacaa agaaacaaaa acagcattta atgaagaagc     420
   tacgaaattc aaagcaaaag ttaatacatt cgtaactgaa ttcaaagaag aaattaaata     480
   aaaataattt aaacaataat attaattaat attatttaaa aataaaaaaa aacattcaaa     540
   atattaaatt ttgaatgttt tttttatttt atattcgaat aaaattgtaa agatgctctt     600
   ataaaatctt taaataaagg atgtggttta aaaggtcttg ataaaaattc a              651
<210> 13
   <211> 868
   <212> DNA
   <213> 16SrV (Elm yellows group)
<400> 13
   atcaaattct tcataaaatt tttaataaaa ttaaataatt taaaatataa caaatataat      60
   aaaatatgtt ataatatttg tattgaatct aagaaaagat ttaataaaat gtatttacat     120
   tttaaatact gtatattaaa taccgtataa attataattt taatgagagg aaagaagaaa     180
   atgcaacaaa aagaaagttt tttaaaaact aaaaaaggca aaatagttgt tggttcagtt     240
   ggttcagttg ttggtgtttt agtattatat ttaataattg cttattttgc gtcactttat     300
   ccatttaata caacaattac tgagaaagat ttaacaacat ttaaaataga tgtagaagaa     360
   ttagtagcag atggaaaaga aacagatgct aaagcagaca ataacaaaat agtagaagca     420
   gtagaaaaaa aaattaacgc tttgaaaaca caagtggaaa aagttaataa aaaaagagct     480
   gacaacaaaa aaattaaaac agaaaaagta gacactttaa aaacagaagc tggcaaatta     540
   aaagacaaag aaacaaaaat agcatttaaa gaagaagcta cgaaattcaa aacaaaagtt     600
   aatacattcg taactgaatt caaagaagaa attaaagaaa aataatttaa aaaataatat     660
   taattaatat tatttaaaaa taaaaaaaac attcaaaatt taatattttg aatgtttttt     720
   ttattttata ttcgaataaa attgtaaaga tgctcttata aagtctttaa ataaaggatg     780
   tggtttaaaa ggtcttgata aaaattcaga atgaaattga actcctataa accaaatatg     840
   tttttttaat tctataactt caggtaat                                        868
<210> 14
   <211> 723
   <212> DNA
   <213> 16SrV (Elm yellows group)
<400> 14
   aatttaaaaa tagaaaataa cgataaagca gataaaattc ttcataaaat ttttaataaa      60
   attaaataat ttaaaatata acaaatataa taaaatatgt tataatattt gtattgaatc     120
   taagaaaaga tttaataaaa tgtatttaca ttttaaatac tgtatattaa ataccgtata     180
   aattataatt ttaatgagag gaaagaagaa aatgcaacaa aaagaaagtt ttttaaaaac     240
   taaaaaaggc aaaatagttg ttggttcagt tggttcagtt ggtggtgttt tagtattata     300
   tttaataatt gcttattttg cgtcacttta tccatttaat acaacaatta ctgagaaaga     360
   tttaacaaaa tttaaaacag atgtagaaga attagtagca gatggaaaag aaacagatgc     420
   taaagtagac aataacaaaa tagtagaagc agtagaaaaa aaaattaacg ctttgaaaga     480
   acaagtggaa aaagttaata aaaaaagagc tgacaacaaa aaaattaaaa cagaaaaagt     540
   agacacttta aaaacagaag ctggcaaatt aaaagacaaa gaaacaaaaa tagcatttaa     600
   agaagaagct acgaaattca aaacaaaagt taatacattc gtaactgaat tccaagaaga     660
   aattaaagaa aaataattta aaaaataata ttaattaata ttatttaaaa ataaaaaaaa     720
   cat                                                                   723
<210> 15
   <211> 896
   <212> DNA
   <213> 16SrV (Elm yellows group)
<400> 15
   taggccacga aaataattta aaaatagaaa ataacgataa agcagataaa attcttcata      60
   aaatttttaa taaaattaaa taatttaaaa tataacaaat ataataaaat atgttataat     120
   atttgtattg aatctaagaa aagatttaat aaaatgtatt tacattttaa atactgtata     180
   ttaaataccg tataaattat aattttaatg agaggaaaga agaaaatgca acaaaaagaa     240
   agttttttaa aaactaaaaa aggcaaaata gttgttggtt cagttggttc agttgttggt     300
   gttttagtat tatttttaat aattgcttat tttgcgtcat tttggccatt tagtaaaaaa     360
   attactaata aagatttaga aacatttaaa acagatgtag aaaaattagt agcagatgaa     420
   aaagcaacag atgttaaaac agacaataac aaaatagtag aagcagtaga aaacaaaatt     480
   aacgctttga aagcacaagt ggaacaagtt aatgaaaaaa gagctgacaa caaaaaaatt     540
   aaaacagaaa aaatagacac tttaaaaaca gaagctgtaa aattaaaaga caaagaaaaa     600
   acagcattta aagaagaagc tacgaaattc aaaacaaaag ttaatacatt cgtaactgaa     660
   ttcaaagaag aaattaaaga aaaataattt aaaaaataat attaattaat attatttaaa     720
   aataaaaaaa acattcaaaa tttaatattt tgaatgtttt ttttatttta tattcgaata     780
   aaattgtaaa gatgctctta taaagtcttt aaataaagga tgtggtttaa aaggtcttga     840
   taaaaattca gaatgaaatt gaactcctat aaaccaaata tgttttttta attcta         896
<210> 16
   <211> 840
   <212> DNA
   <213> 16SrV (Elm yellows group)
<400> 16
   aataatttaa aaaatagaaa ataacgataa agcagataaa attcttcata aaatttttaa      60
   taaaattaaa taatttaaaa tataacatat ataataaaat atgttataat atttgtattg     120
   aatctaagaa aagatttaat aaaatgtatt tacattttaa atactgtata ttaaataccg     180
   tataaattat aatttaatga gaggaaagaa gaaaatgcaa caaaaagaaa gttttttaaa     240
   aactaaaaaa ggcaaaatag ttgttggttc agttggttca gttattggtg ctttagtatt     300
   atatttaata attgcttatg ttatgaaatg gattccattt agggtaaaaa ttactaatga     360
   agatttagaa acatttaaaa caaatgtaga aaaattagta gcagatgaca aagcaaaaga     420
   agctaaagca gacaatgaca aaatagtaga agaagtagaa aacaaaatta acgctttgaa     480
   agcacaagtg gatcaagtta ataaaaaaag agcttcagac aaaaaaattg gcacacaaaa     540
   aatagaagac ttaaaaacag aagctggcaa attaaaagac aaagaaaaag cagcatttaa     600
   aacagcagct acagaattca aaacaaaagt cggtactttc gtaactagtt tcaaagaaga     660
   cattagagaa aaataattta aacaataata ttaattaata ttatttaaaa ataaaaaaaa     720
   acattcaaaa tttaatattt tgaatgtttt ttttatttta tatttgaata aaattgtaaa     780
   gatgctctta taaatcttta aataaaggat gtggtttaaa aggtcttgat aaaaattcag     840
<210> 17
   <211> 739
   <212> DNA
   <213> 16SrV (Elm yellows group)
<400> 17
   aaataattta aaaatagaaa ataatgataa agcagataaa attcttcata aaatttttaa      60
   taaaattaaa taatttaaaa tataacatat ataataaaat atgttataat atttgtattg     120
   aatctaagaa aagatttaat aaaatgtatt tacattttaa atactgtata ttaaataccg     180
   tataaattat aatttaatga gaggaaagaa gaaaatgcaa caaaaagaaa gttttttaaa    240
   aactaaaaaa ggcaaaatag ttgttggttc agttggttca gttgttggtg ttttagtatt     300
   atttttaata attgcttatt ttgcgtcaat ttggccattt agtacaaaaa ttactgataa     360
   agatttagaa acatttaaaa cagatgtaga aaaattagta gcagatgaaa aagcaaaaga     420
   tgctaaagca gacaatgaca aaatagtaga agaagtagaa aaaaaaatta acgctttgaa     480
   agcacaagtg gataaagtta ataaaaaaag agcttcagac aaaaaaattg gcacacaaaa     540
   aatagaagac ttaaaaacag aagctggcaa attaaaagac aaagacaaag cagcatttaa     600
   aacagcagct acagaattca aaacaaaagt cggtactttc gtaactagtt tcggagaaga     660
   cattaaagaa aaataattta aacaataata ttaattaata ttatttaaaa ataaaaaaaa     720
   acattcaaaa tattaaatt                                                   739
<210> 18
   <211> 789
   <212> DNA
   <213> 16SrV (Elm yellows group)
<400> 18
   aaataattta aaaatagaaa ataacgataa agcagataaa attctttcat aaaattttta      60
   ataaaattaa ataatttaaa atataacata tataataaaa tatgttataa tatttgtatt     120
   gaatctaaga aaagatttaa taaaatgtat ttacatttta aatactgtat attaaatacc     180
   gtataaatta taatttaatg agaggaaaga agaaaatgca acaaaaagaa agttttttaa     240
   aaactaaaaa aggcaaaata gttgttggtt cagttggttc agttattggt gttttagtat     300
   tatatttaat aattgcttat attatgaaat ggattccatt tcgtatatca attaatgata     360
   cagatttaac aacatttaag acagaagtag aaaaattagt agcagatgac aaagcaaaag     420
   atgataaaac agaaaataac aaaatattag aatcagtaga aaacaaaatt aacgctttga     480
   aaaaacaagt ggaaaaagtt aatgaaaaaa gagctgacaa caaaaaaatt aaaacagaaa     540
   aaatagacac tttaaaaaca gaagctgtca aattaaaaga caaagaaaca aaaatagcat     600
   ttaatgcaga agctacgaaa ttcaaaacaa acgttgatag attcgtaact gaattcaaag     660
   aagaaattaa agaaaaataa tttaaacaat aatattaatt aatattattt aaaaataaaa     720
   aaaacattca aaatttaata ttttgaatgt ttttttattt ttatatttga ataaaatgta     780
   aagatgcta                                                             789
<210> 19
   <211> 783
   <212> DNA
   <213> 16SrV (Elm yellows group)
<400> 19
   ataatttaaa aatagaaaat aatgataaag cagataaaat tcttcataaa atttgtaata       60
   aagttaaata atttaaaata taacaaatat aataaaatat gttataatat ttgtattgaa      120
   tctaagaaaa gatttaataa aatgtattta cattttaaat actgtatatt aaataccgta      180
   taaattataa ttttaatggg aggaaagaag aaaatgcaac aaaaagaaag ttttttaaaa      240
   actaaaaaag gcaaaatagt tgttggttca gttggttcag ttgttggtgt tttagtatta      300
   tttttaataa ttgcttattt tgcgtcaatt tggccattta gtacaaaaat taatgataaa      360
   gatttagaaa catttaaaac agatgtagaa aaattagtag cagatgacaa agcaggttct      420
   aaaacagaaa ataacacaac agcagaaaaa gtagaagaaa aaatcaaaga agtgaaaaag      480
   ctagtagaca aagttaatga aaaaagagct tcagacaaaa aaattgacac aaaaaaaata      540
   gaagacttaa aaacagaagc tgccaaatta aaagaaaaag aaaaagaagc atttaaaaca      600
   gcagctgcag aattcaaaac aaaagtcggt agtttcgtaa ctagtttcaa agaagacatt      660
   aaagaaaaat aatttaaaca ataatattaa ttaatattat ttaaaaataa aaaaaacatt      720
   caaaatttaa tattttgaat gtttttttta ttttatattg aataaaatgt aaagaagctc       780
   ttg                                                                     783
<210> 20
   <211> 756
   <212> DNA
   <213> 16SrV (Elm yellows group)
<400> 20
   aaaaagattt ttaaaaatgt atttacattt taaatactgt atattaaata ccgtataaat       60
   tataatttta atgagaggaa agaagaaaat gcaacaaaaa gaaagttttt taaaaactaa 120
   aaaaggcaaa atagttgttg gttcagttgg ttcagttgtt ggtgttttag tattattttt 180
   aataattgct tattttgcgt caatttggcc atttagtaca aaaattactg ataaagattt 240
   agaaacattt aaaacagatg tagaaaaatt agtagcagat gacaaagcag gttctaaaac 300
   agaaaataac acaacagcag aaaaagtaga agaaaaaatc aaagaagtga aaaagctagt 360
   agacaaagtt aatgaaaaaa gagcttcaga caaaaaaatt gacacaaaaa aaatagaaga 420
   cttaaaaaca gaagctgcca aattaaaaga aaaagaaaaa gaagcattta aaacagcagc 480
   tgcagaattc aaaacaaaag tcggtagttt cgtaactagt ttcaaagaag acattaaaga 540
   aaaataattt aaacaataat attaattaat attatttaaa aataaaaaaa acattcaaaa 600
   tttaatattt tgaatgtttt ttttatttta tatttgaata aaattgtaaa gatgctctta 660
   taaaatcttt aaataaagga tgtggtttaa aaggtcttga taaaaattca gaatgaaatt 720
   gaacccctat aaaccaaata tgttttttta attcta 756
<210> 21
   <211> 580
   <212> DNA
   <213> 16SrV (Elm yellows group)
<400> 21
   ttaaataccg tataaattat aattttaatg agaggaaaga agaaaatgca acaaaaagaa 60
   agttttttaa aaactaaaaa aggcaaaata gttgttggtt cagttggttc agttgttggt 120
   gttttagtat tatttttaat aattgcttat tttgcgtcat tttggccatt taatccaaca 180
   attactgaga aagatttaac aacatttaaa acagatgtag aaaaattagt agcagatgaa 240
   aaagcaacag atgttgaagc agacaatgac aaaatagtat tagatgtaga aaaaaaaatt 300
   aacgctttaa aaacacaagt agaagaagtt aataaaaaaa gagctgacga caaaaaaatt 360
   aaaacagaca aaatagacaa tttaaaaata gaagctgaca aattaaaagg caaaaaaaca 420
   aaagacacat ttaaagatga agctaagaaa ttcaaagcaa gcgttgatac attcgtaact 480
   ggatacaaaa cagacattaa agaataataa tttaaaaaat gatattaatt aatattattt 540
   aaaaataaaa aaaaacattc aaaatttaat attttgaatg 580
<210> 22
   <211> 801
   <212> DNA
   <213> 16SrV (Elm yellows group)
<400> 22
   aaaatagaaa ataacgataa agcagataaa attcttcata aaatttttaa taaaattaaa      60
   taatttaaaa tataacaaat ataataaaat atgttataat atttgtattg aatctaagaa     120
   aagatttaat aaaatgtatt tacattttaa atactgtata ttaaataccg tataaattat     180
   aattttaatg aaaggaaaga agaaaatgca acaaaaagaa agttttttaa aaactaaaaa      240
   aggcaaaata gttgttggtt cagttggttc agttgttggt gttttagtat tatttttaat     300
   aattgcttat attgtgccat tttggccatt tagtacaaaa attactaata aagatttaga     360
   aacatttaaa aaagatgtag aaaaattagt agcagatgaa aaagcaaaag atgataaaac     420
   agacaataac aaaatagtag aagcagtaga aaacaaaatt aacgctttga aaacacaagt     480
   gacaacagtt aatgaaaaaa gagctgacaa caaaaaaatt aaaacagaaa aaatagacac     540
   tttaaaaaca gaagctgtca aattaaaaga caaagaaaca aaaacagcat ttaatgaaga     600
   agctacgaaa ttcaaagcaa aagttaatac attcgtaact gaattcaaag aagaaattaa     660
   ataaaaataa tttaaacaat aatattaatt aatattattt aaaaataaaa aaaaacattc     720
   aaaatattaa attttgaatg ttttttttat tttatattcg aataaaattg taaagatgct     780
   cttataaaat ctttaaataa a                                               801
<210> 23
   <211> 675
   <212> DNA
   <213> 16SrV (Elm yellows group)
<400> 23
   atttttaata aaattaarta atttaaaata taacatmtat aataaaatat gttataatat      60
   ttgtattgrr kctaagaaaa gatttaataa aatgtattta cmttttaaat actgtataty     120
   aaataccgta yaaattataa tttaatgggg ggaaagraga aaatgcaaca aaaagaaagt     180
   tttttaaaaa ctaaaaaagg caaaatagtt gttggttcag ttggttcagt tgttggkgtt     240
   ttagtattat ttttaataat tgcttakttk gsgkcaattt ggccatttag tacaaaaatt     300
   actaataaag atttagaaaa atttaaaaca gatgtagaaa aattagtaga aagtgaacaa     360
   gcaaaagatt cgaaaacaga caataacaaa atagtwgaaa cagtagaaaa aaaaattaac     420
   gctttgaaaa cacaagtgac aacrgttaat gaaaaaagag ctgacaacaa aaaaattaaa     480
   acagaaaaaa tagacccttt aaaaacagaa gctgccaaat taaaagacaa araaacaaaa     540
   acagcattta akgaagaagc tacgaaattc aaagcaaaag ttaatacatt cgtaactgaa     600
   ttcaaagaag aaattaaata aaaataattt aaacaataat attaattaat attatttaaa     660
   aataaaaaaa aacat                                                      675
<210> 24
   <211> 883
   <212> DNA
   <213> 16SrV (Elm yellows group)
<400> 24
   aaataattta aaaatagaaa ataacgataa agcagataaa attcttcata aaatttttaa      60
   taaaattaaa taatttaaaa tataacaaat ataataaaat atgttataat atttgtattg     120
   aatctaagaa aagatttaat aaaatgtatt tacattttaa atactgtata ttaaataccg     180
   tataaattat aattttaatg agaggaaaga agaaaatgca acaaaaagaa agttttttaa     240
   aaactaaaaa aggcaaaata gttgttggtt cagttggttc agttgttggt gttttagtat     300
   tatttttaat aattgcttat tttgcgtcat tttggccatt tagtaaaaaa attactaata     360
   aagatttaga aacatttaaa acagatgtag aaaaattagt agcagatgaa aaagcaacag     420
   atgttaaaac agacaataac aaaatagtag aagcagtaga aaacaaaatt aacgctttga     480
   aagcacaagt ggaacaagtt aatgaaaaaa gagctgacaa caaaaaaatt aaaacagaaa     540
   aaatagacac tttaaaaaca gaagctgtaa aattaaaaga caaagaaaaa acagcattta     600
   aagaagaagc tacgaaattc aaaacaaaag ttaatacatt cgtaactgaa ttcaaagaag     660
   aaattaaaga aaaataattt aaaaaataat attaattaat attatttaaa aataaaaaaa     720
   acattcaaaa tttaatattt tgaatgtttt ttttatttta tattcgaata aaattgtaaa     780
   gatgctctta taaagtcttt aaataaagga tgtggtttaa aaggtcttga taaaaattca     840
   gaatgaaatt gaactcctat aaaccaaata tgttttttta att                       883
<210> 25
   <211> 634
   <212> DNA
   <213> 16SrV (Elm yellows group)
<400> 25
   atttacattt taaatactgt atattaaata ccgtataaat tataatttaa tgagaggaaa      60
   gaagaaaatg caacaaaaag aaagtttttt aaaaactaaa aaaggcaaaa tagttgttgg     120
   ttcagttggt tcagttgttg gtgttttagt attattttta ataattgctt attttgcgtc     180
   aatttggcca tttagtacaa aaattactaa taaagattta gaaaaattta aaacagatgt     240
   agaaaaatta gtaaaagatg aacaagcaaa agattcgaaa acagacaata acaaaatagt     300
   agaaacagta gaaaaaaaaa ttaacgattt gaaaacacaa gtgacaacag ttaatgaaaa     360
   aagagctgac aacaaaaaaa ttaaaacaga aaaaatagac actttaaaaa cagaagctgc     420
   caaattaaaa gacaaagaaa caaaaacagc atttaatgaa gaagctacga aattcaaagc     480
   aaaagttaat acattcgtaa ctgaattcaa agaagaaatt aaataaaaat aatttaaaca     540
   ataatattaa ttaatattat ttaaaaataa aaaaaaacat tcaaaatatt aaattttgaa     600
   tgtttttttt attttatatt cgaataaaac gtag                                 634
<210> 26
   <211> 698
   <212> DNA
   <213> 16SrV (Elm yellows group)
<400> 26
   taaaattaaa taatttaaaa tataacatat ataataaaat atgttataat atttgtattg      60
   aatctaagaa aagatttaat aaaatgtatt tacattttaa atactgtata ttaaataccg     120
   tataaattat aatttaatga gaggaaagaa gaaaatgcaa caaaaagaaa gtttttaaaa     180
   aactaaaaaa ggcaaaatag ttgttggttc agttggttca gttgttggtg ttttagtatt     240
   atttttaata attgcttatt ttgcgtcaat ttggccattt agtacaaaaa ttactaataa     300
   agatttagaa aaatttaaaa cagatgtaga aaaattagta gaagatgaac aagcaaaaga     360
   ttcgaaaaca gacaataaca aaatagtaga aacagtagaa aaaaaaatta acgctttgaa     420
   aacacaagtg acaacagtta atgaaaaaag agctgacaac aaaaaaatta aaacagaaaa     480
   aatagacact ttaaaaacag aagctgccaa attaaaagac aaagaaacaa aaacagcatt     540
   taatgaagaa gctacgaaat tcaaagcaaa agttaatgca ttcgtaactg aattcaaaga     600
   agaaattaaa taaaaataat ttaaacaata atattaatta atattattta aaaataaaaa     660
   aaaacattca aaatattaaa ttttgaatgt tctttttt                             698
<210> 27
   <211> 703
   <212> DNA
   <213> 16SrV (Elm yellows group)
<400> 27
   ttcttcataa aatttttaat aaaattaaat aatttaaaat ataacaaata taataaaata 60
   tgttataata tttgtattga atctaagaaa agatttaata aaatgtattt acattttaaa 120
   tactgtatat taaataccgt ataaattata attttaatga gaggaaagaa gaaaatgcaa 180
   caaaaagaaa gttttttaaa aactaaaaaa ggcaaaatag ttgttggttc agttggttca 240
   gttgttggtg ttttagtatt atttttaata attgcttatt ttgcgtcatt gtggccattt 300
   agtacaaaaa ttactaataa agatttagaa acatttcaaa aagatgtaga aaaattagta 360
   gcagatgaaa aagcaacaga tgttaaaaca gacaataaca aaatagtaga agcagtagaa 420
   aacaaaatta acgctttgaa aacacaagtg gacaaagtta ataaaaaaag agctgacaac 480
   aaaaaaatta aaacagaaaa aatagacact ttaaaaacag aagctgcaaa attaaaagac 540
   aaagaaaaaa cagcatttaa agaagaagct acgaaattca aaacaaacgt tgataaattc 600
   gtaactgaat tcaaagaaga aattaaagaa aaataattta aacaataata ttaattaata 660
   ttatttaaaa ataaaaaaaa cattcaaaat ttaatatttt gaa 703
<210> 28
   <211> 858
   <212> DNA
   <213> 16SrV (Elm yellows group)
<400> 28
   aaataattta aaaatagaaa ataacgataa agcagataaa attcttcata aaatttttaa 60
   taaaattaaa taatttaaaa tataacatat ataataaaat atgttataat atttgtattg 120
   aatctaagaa aagatttaat aaaatgtatt tacattttaa atactgtata ttaaataccg 180
   tataaattat aatttaatga gaggaaagaa gaaaatgcaa caaaaagaaa gttttttaaa 240
   aactaaaaaa ggcaaaatag ttgttggttc agttggttca gttattggtg ttttagtatt 300
   atatttaata attgcttata ttatgaaatg gattccattt cgtatatcaa ttaataatac 360
   agatttaaca acatttaaga cagaagtaga aaaattagta gcagatgaca aagcaacaga 420
   tgttaaagca gacaataaca aaatagtaga agcagtagaa gaaaaaatta cagctttgaa 480
   acgacaagtg ggcaaagtta atgaaaaaag agctgacgac aaaaaaatta aaacagaaaa 540
   aatagacact ttaaaaacag acgctggcaa attaaaagac aaagaaacaa aaacagcatt 600
   taaagaagaa gctgcgaaat tcaaaacaag cgttaataaa ttcgtaaatg aattcaaaga 660
   agaaattaat taatattatt taaaaataaa aaaaacattc aaaatttaat attttgaatg 720
   ttttttttat tttatatttg aataaaattg taaagatgct cttataaagt ctttaaataa 780
   agggtgtggt ttaaaaggtc ttgataaaaa ttcagaatga aattgaactc ctataaacca 840
   aatatgtttt tttaattc 858
<210> 29
   <211> 786
   <212> DNA
   <213> 16SrV (Elm yellows group)
<400> 29
   aataaaatta aataatttaa aatataacat atataataaa atatgttata atatttgtat      60
   tgaatctaag aaaagattta ataaaatgta tttacatttt aaatactgta tattaaatac     120
   cgtataaatt ataatttaat gagaggaaag aagaaaatgc aacaaaaaga aagtttttta     180
   aaaactaaaa aaggcaaaat agttgttggt tcagttggtt cagttgttgg tgttttagta     240
   ttatttttaa taattgctta ttttgcgtca atttggccat ttagtacaaa aattactaat     300
   aaagatttag aaaaatttaa aacagatgta gaaaaattag tagaagataa acaagcaaaa     360
   gattcgaaaa cagacaataa caaaatagta gaaacagtag aaaaaaaaat taacgctttg     420
   aaaacacaag tgacaacagt taatgaaaaa agagctgaca acaaaaaaat taaaacagaa     480
   aaaatagaca ctttaaaaac agaagctgcc aaattaaaag acaaagaaac aaaaacagca     540
   tttaatgaag aagctacgaa attcaaagca aaagttaatg cattcgtaac tgaattcaaa     600
   gaagaaatta aataaaaata atttaaacaa taatattaat taatattatt taaaaataaa     660
   aaaaaacatt caaaatatta aattttgaat gttttttttt attttatatt cgaataaaat     720
   tgtaaagatg ctcttataaa atctttaaat aaaggatgcg gtttaaaagg tcttgataaa     780
   aattca                                                                786
<210> 30
   <211> 665
   <212> DNA
   <213> 16SrV (Elm yellows group)
<400> 30
   tattgaatct aagaaaagat ttaataaaat gtatttacat tttaaatact gtatattaaa      60
   taccgtataa attataattt aatgagagga aagaagaaaa tgcaacaaaa agaaagtttt     120
   ttaaaaacta aaaaaggcaa aatagttgtt ggttcagttg gttcagttgt tggtgtttta     180
   gtattatttt taataattgc ttattttgcg tcaatttggc catttagtac aaaaattact     240
   aataaagatt tagaaaaatt taaaacagat gtagaaaaat tagtagaaga tgaaaaagca     300
   aaagattcga aaacagacaa taacaaaata gtagaaacag tagaaaaaaa aattaacgct     360
   ttgaaaacac aagtgacaac agttaatgaa aaaagagctg acaacaaaaa aattaaaaca     420
   gaaaaaatag acactttaaa aacagaagct gccaaattaa aagacaaaga aacaaaaaca     480
   gcatttaatg aagaagctac gaaattcaaa gcaaaagtta atacattcgt aactgaattc     540
   aaagaagaaa ttaaataaaa ataatttaaa caataatatt aattaatatt atttaaaaat     600
   aaaaaaaaca ttcaaaatat taaattttga atgttttttt tattttatat tcgaataaaa     660
   tgtaa                                                                 665
<210> 31
   <211> 666
   <212> DNA
   <213> 16SrV (Elm yellows group)
<400> 31
   atgttataat atttgtattg aatctaagaa aagatttaat aaaatgtatt tacattttaa      60
   atactgtata ttaaataccg tataaattat aatttaatga gaggaaagaa gaaaatgcaa     120
   caaaaagaaa gttttttaaa aactaaaaaa ggcaaaatag ttgttggttc agttggttca     180
   gttgttggtg ttttagtatt atttttaata attgcttatt ttgcgtcaat ttggccattt     240
   agtacaaaaa ttactaataa agatttagaa aaatttaaaa cagatgtaga aaaattagta     300
   gaagatgaac aagcaaaaga ttcgaaaaca gacaataaca aaatagtaga aacagtagaa     360
   aaaaaaatta acgctttgaa aacacaagtg acaacagtta atgaaaaaag agctgacaac     420
   aaaaaaatta aaacagaaaa aatagacact ttaaaaacag aagctgccaa attaaaagac     480
   aaagaaacaa aaacagcatt taatgaagaa gctacgaaat tcaaagcaaa agttaataca     540
   ttcgtaactg aattcaaaga agaaattaaa taaaaataat ttaaacaata atattaatta     600
   atattattta aaaataaaaa aaaacattca aaatattaaa ttttgaatgt ttttttttta     660
   ttttat                                                                666
<210> 32
   <211> 885
   <212> DNA
   <213> 16SrV (Elm yellows group)
<400> 32
   aataatttaa aaatagaaaa taacgataaa gcagataaaa ttcttcataa aatttttaat      60
   aaaattaaat aatttaaaat ataacaaata taataaaata tgttataata tttgtattga     120
   atctaagaaa agatttaata aaatgtattt acattttaaa tactgtatat taaataccgt     180
   ataaattata attttaatga gaggaaagaa gaaaatgcaa caaaaagaaa gttttttaaa    240
   aactaaaaaa ggcaaaatag ttgttggttc agttggttca gttgttggtg ttttagtatt     300
   atttttaata attgcttatt ttgcgtcaat ttggccattt agtacaaaaa ttactaataa     360
   agatttagaa acatttaaag aagatgtaga aaaattagta gcagatgaaa aagcaacaaa     420
   tgttaaaaca gacaataaca aaatagtaga agaagtagaa aaaaaaatta acgctttgaa     480
   aacacaagtg gaaaaagtta ataaaaaaag agctgacaac aaaaaaatta aaacagaaaa     540
   aatagacact ttaaaaacag aagctgtcaa attaaaagaa aaagaaacaa aagacacatt     600
   taaaagtgaa gctagtagat tcaaagcaag cgttgataca ttcgtaactg aattcaaaga     660
   agaaattaaa gaaaaataat ttaaacaata atattaatta atattattta aaaataaaaa     720
   aaacattcaa aatattaaat tttgaatgtt ttttttattt tatattcgaa taaaatgtaa     780
   agatgctctt ataaaatctt taaataaagg atgtggttta aaaggtcttg ataaaaattc     840
   agaatgaatt tgaactccta taaaccaaat atgttttttt aattc                     885
<210> 33
   <211> 749
   <212> DNA
   <213> 16SrV (Elm yellows group)
<400> 33
   cgataaagca gataaaattc ttcataaaat ttttaataaa attaaataat ttaaaatata      60
   acaaatataa taaaatatgt tataatattt gtattgaatc taagaaaaga tttaataaaa     120
   tgtatttaca ttttaaatac tgtatattaa ataccgtata aattataatt taatgagagg     180
   aaagaagaaa atgcaacaaa aagaaagttt tttaaaaact aaaaaaggca aaatagttgt     240
   tggttcagtt ggttcagtta ttggtgcttt agtattatat ttaataattg cttatattat     300
   gaaatggatt ccatttaggg aaaaaattac tgataaagat ttagaaacat ttaaaacaga     360
   tgtagaaaaa ttagtagcag atgaaaaagc aatggatact aaagcagaca ataacaaaat     420
   agtagaagca gtagaaaaaa aaattaacgc tttgaaagca caagtggata aagttaatga     480
   aaaaagagct tcagacaaaa aaattggcac aaaaaaaata gaagacttaa aaacagaagc     540
   tggcaaatta aaagacaaag aaaaagaagc atttaaaaca gtagctgcag aattcaaaac 600
   aaaagtcggt gatttcgtaa ctagtttcaa agaagacatt aaagaaaaat aatttaaaca 660
   ataatattaa ttaatattat ttaaaaataa aaaaaacatt caaaatttaa tattttgaat 720
   gtttttttta ttttatattc gaataaaat 749
<210> 34
   <211> 882
   <212> DNA
   <213> 16SrV (Elm yellows group)
<400> 34
   aaataattta aaaatagaaa ataacgataa agcagataaa attcttcata aaatttttaa 60
   taaaattaaa taatttaaaa tataacaaat ataataaaat atgttataat atttgtattg 120
   aatctaagaa aagatttaat aaaatgtatt tacattttaa atactgtata ttaaataccg 180
   tataaattat aatttaatga gaggaaagaa gaaaatgcaa caaaaagaaa gttttttaaa 240
   aactaaaaaa ggcaaaatag ttgttggttc agttggttca gttattggtg ctttagtatt 300
   atatttaata attgcttata ttatgaaatg gattccattt agggtaaaaa ttactgataa 360
   agatttagaa acatttaaaa cagatgtaga aaaattagta gcagatgaaa aagcaacaga 420
   tactaaagca gacaataaca aaatagtaga agcagtagaa aaaaaaatta acgctttgaa 480
   agcacaagtg gatcaagtta atgaaaaaag agcttcagac aaaaaaattg gcacaaaaaa 540
   aatagaagac ttaaaaacag aagctggcaa attaaaagac aaagataaag aagcatttaa 600
   aacagtagct gcagaattca aaacaaaagt cggtgatttc gtaactagtt tcaaagaaga 660
   cattaaagaa aaataattta aacaataata ttaattaata ttatttaaaa ataaaaaaaa 720
   cattcaaaat ttaatatttt gaatgttttt tttattttat attcgaataa aattgtaaag 780
   atgctcttat aaagtcttta aataaaggat gtggtttaaa aggtcttgat aaaaattcag 840
   aatgaaattg aactcctata aaccaaatat gtttttttaa tt 882
<210> 35
   <211> 743
   <212> DNA
   <213> 16SrV (Elm yellows group)
<400> 35
   ataaaatttt taataaaata aaataattta aaatataaca tatataataa aatatgttat      60
   aatatttgta ttgaatctaa gaaaagattt aataaaatgt atttacattt taaatactgt     120
   atattaaata ccgtataaat tataatttaa tgagaggaaa gaagaaaatg caacaaaaag     180
   aaagtttttt aaaaactaaa aaaggcaaaa tagttgttgg ttcagttggt tcagttattg     240
   gtgctttagt attatattta ataattgctt atattatgaa atggattcca tttagggaaa     300
   aaattactcc ggaagatgca gaaagtttaa aaacaaaaat agaatcatta gtagcagata     360
   ctgaagcaaa aacaactgca acagacaatg acaaaatata tgacaaagta aaaaaagcaa     420
   ttgaagcttt tgaatcaaaa gtaacagcag ttaatgaaaa aagagctaca gaaaaaaaaa     480
   ttgatactac tgaaataaca aatttaaaaa aagaagttga aaatttaaaa aatcaagata     540
   ctcaagacaa atttaaagca gcagcaaaaa ttttcaaaga caaagttgct atatcaataa     600
   caaatttaaa aacagtaatt acagaaaaaa acaaataata atttaaacaa taatattaat     660
   taatattatt taaaaataaa aaaaaacatt caaaatatta aattttgaat gttttttttt     720
   attttatatt cgaataaaat gta                                             743
<210> 36
   <211> 632
   <212> DNA
   <213> 16SrV (Elm yellows group)
<400> 36
   gtatttacat tttaaatact gtatattaaa taccgtataa attataattt aatgagagga      60
   aagaagaaaa tgcaacaaaa agaaagtttt ttaaaaacta aaaaaggcaa aatagttgtt     120
   ggttcagttg gttcagttat tggtgcttta gtattatatt taataattgc ttatattatg     180
   aaatggattc catttaggga aaaaattact ccggaagatg cagaaagttt aaaaacaaaa     240
   atagaatcat tagtagcaaa tactgaagca aaaacaactg tagaagagaa tgacaaaata     300
   tatgacaaag taaaaaaagc aattgaagct tttgaatcaa aagtaaaagc agttaatgaa     360
   aaaagagcta cagaaaaaaa aattgatact actgaaataa caaatttaaa aacagcagtt     420
   gaaggtttaa aaaataaaaa tactcaagaa aattttaaaa cagcagcaaa agatttcaaa     480
   gacaaagttg ctacatcaat aacaaattta aaaacagtaa ttacagaaaa aaacaaataa     540
   taatttaaac aataatatta attaatatta tttaaaaata aaaaaaacat tcaaaatatt     600
   aaattttgaa tgtttttttt attttatatt cg                                   632
<210> 37
   <211> 885
   <212> DNA
   <213> 16SrV (Elm yellows group)
<400> 37
   aataatttaa aaatagaaaa taatgataaa gcagataaaa ttcttcataa aatttttaat      60
   aaaattaaat aatttaaaat ataacatata taataaaata tgttataata tttgtattga     120
   atctaagaaa agatttaata aaatgtattt acattttaaa tactgtatat taaataccgt     180
   ataaattata atttaatgag aggaaagaag aaaatgcaac aaaaagaaag ttttttaaaa     240
   actaaaaaag gcaaaatagt tgttggttca gttattggtg ctttagtatt atatttaata     300
   attgcttata ttatgaaatg gattccattt agggaaaaaa ttactccgga agatgcagaa     360
   agtttaaaaa caaaaataga atcattagta gcaaatactg aagcaaaaac aactgtagaa     420
   gagaatgaca aaatatatga caaagtaaaa aaagcaattg aagcttttga atcaaaagta     480
   aaagcagtta atgaaaaaag agctacagaa aaaaaaattg atactactga aataacaaat     540
   ttaaaaacag cagttgaagg tttaaaaaat aaaaatactc aagaaaattt taaaacagca     600
   gcaaaagatt tcaaagacaa agttgctaca tcaataacaa atttaaaaac agtaattaca     660
   gaaaaaaaca aataataatt taaacaataa tattaattaa tattatttaa aaataaaaaa     720
   aacattcaaa atattaaatt ttgaatgttt tttttatttt atattcgaat aaaattgtaa     780
   agatgctctt ataaagtctt taaataaagg gtgtggttta aaaggtcttg ataaaaattc     840
   agaatgaaat tgaactccta taaaccaaat atgttttttt aattc                     885
<210> 38
   <211> 646
   <212> DNA
   <213> 16SrV (Elm yellows group)
<400> 38
   agatttaata aaatgtattt acattttaaa tactgtatat taaataccgt ataaattata      60
   atttaatgag aggaaagaag aaaatgcaac aaaaagaaag ttttttaaaa actaaaaaag     120
   gcaaaatagt tgttggttca gttggttcag ttattggtgc tttagtatta tatttaataa     180
   ttgcttatat tatgaaatgg attccattta gggaaaaaat tactgataaa gatttagaaa     240
   catttaaaac agatgtagaa aaattagtag cagatgaaaa agcaatggat actaaagcag     300
   aaaataacaa aatagtagaa gcagtagaaa aaaaaattaa cgctttgaaa gcacaagtgg     360
   ataaagttaa tgaaaaaaga gcttcagaca aaaaaattgg cacaaaaaaa atagaagact     420
   taaaaacaga agctggcaaa ttaaaagaca aagaaaaaga agcatttaaa acagtagctg     480
   cagaattcaa aacaaaagtc ggtgatttcg taactagttt caaagaagac attaaagaaa     540
   aataatttaa acaataatat taattaatat tatttaaaaa taaaaaaaac attcaaaatt     600
   taatattttg aatgtttttt ttattttata ttcgaataaa attgta                    646
<210> 39
   <211> 777
   <212> DNA
   <213> 16SrV (Elm yellows group)
<400> 39
   aatttaaaaa tagaaaataa cgataaagca gataaaattc ttcataaaat ttttaataaa      60
   attaaataat ttaaaatata acaaatataa taaaatatgt tataatattt gtattgaatc     120
   taagaaaaga tttaataaaa tgtatttaca ttttaaatac tgtatattag ataccgtata     180
   aattataatt ttaatgagag gaaagaagaa aatgcaacca aaagaaagtt ttttaaaaac     240
   taaaaaaggc aaaatagttg ttggttcagt tggttcagtt gttggtgttt tagtattatt     300
   tttaataatt gcttattttg cgtcattttg gccatttaat ccaacaatta ctgagaaaga     360
   tttaacaaca tttaaaacag atgtagaaaa attagtagaa gatggaaaag caacagatgt     420
   taaagcagac aataacaaaa tagtagaagc agtagaaaac aaaattaacg ctttgaaaac     480
   acaagtggaa caagttaata aaaaaagagc tgacaacaaa aaaattaaaa cagaaaaaat     540
   agacacttta aaaacagaag ctgtcaaatt aaaagacaaa gaaacaaaag tagcatttaa     600
   agaagaagct acgaaattca aaacaaaagt tgatacattc gtaactgaat tcaaagaaga     660
   aattaaagaa aaataattta aacaataata ttaattaata ttatttaaaa ataaaaaaaa     720
   cattcaaaat ttaatatttt gaatgttttt tttattttat atttgaataa aattgta        777
<210> 40
   <211> 736
   <212> DNA
   <213> 16SrV (Elm yellows group)
<400> 40
   tgtattgaat ctaagaaaag atttaataaa atgtatttac attttaaata ctgtatatta      60
   aataccgtat aaattataat ttaatgagag gaaagaagaa aatgccacaa aaagaaagtt     120
   ttttaaaaac taaaaaaggc aaaatagttg ttggttcagt tggttcagtt attggtgttt     180
   tagtattata tttaataatt gcttatatta tgaaatggat tccatttcgt atatcaatta     240
   atgatacaga tttaacaaca tttaagacag aagtagaaaa attagtagca gatgacaaag     300
   caaaagatga taaaacagaa aataacaaaa tattagaatc agtagaaaac aaaattaacg     360
   ctttgaaaaa acaagtggaa aaagttaatg aaaaaagagc tgacaacaaa aaaattaaaa     420
   cagaaaaaat agacacttta aaaacagaag ctgtcaaatt aaaagacaaa gaaacaaaaa     480
   tagcatttaa tgcagaagct acgaaattca aaacaaacgt tgatagattc gtaactgaat     540
   tcaaagaaga aattaaagaa aaataattta aacaataata ttaattaata ttatttaaaa     600
   ataaaaaaaa cattcaaaat ttaatatttt gaatgttttt tttattttat atttgaataa     660
   aattgtaaag atgctcttat aaaatcttta aataaaggat gcggtttaaa aggtcttgat     720
   aaaaattcag aatgaa                                                     736
<210> 41
   <211> 156
   <212> PRT
   <213> 16SrV (Elm yellows group)
<400> 41
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
               20                  25                  30
   Ile Ala Tyr Val Met Lys Trp Ile Pro Phe Arg Glu Lys Ile Thr Pro
           35                  40                  45
   Glu Asp Ala Glu Ser Leu Lys Thr Lys Ile Glu Ser Leu Val Ala Asp
       50                  55                  60
   Thr Glu Ala Lys Thr Thr Val Ala Asp Asn Asp Lys Ile Tyr Asp Lys
   65                  70                  75                  80
   Val Lys Lys Ala Ile Glu Ala Phe Glu Ser Lys Val Thr Ala Val Asn
                   85                  90                  95
   Asp Lys Arg Ala Thr Glu Lys Lys Ile Asp Thr Thr Glu Ile Ala Asn
               100                 105                 110
   Leu Lys Thr Ala Val Glu Gly Leu Lys Asn Gln Asp Thr Gln Glu Asn
           115                 120                 125
   Phe Lys Ala Ala Ala Lys Asn Phe Lys Val Lys Val Ala Asn Ser Ile
       130                 135                 140
   Thr Asn Leu Lys Thr Val Ile Thr Glu Lys Asn Lys
   145                 150                 155
<210> 42
   <211> 156
   <212> PRT
   <213> 16SrV (Elm yellows group)
<400> 42
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
               20                  25                  30
   Ile Ala Tyr Val Met Lys Trp Ile Pro Phe Arg Glu Lys Ile Thr Pro
           35                  40                  45
   Glu Asp Ala Glu Ser Leu Lys Thr Lys Ile Glu Ser Leu Val Ala Asp
       50                  55                  60
   Thr Glu Ala Lys Thr Thr Val Ala Asp Asn Asp Lys Ile Tyr Asp Lys
   65                  70                  75                  80
   Val Lys Lys Ala Ile Glu Ala Phe Glu Ser Lys Val Thr Ala Val Asn
                   85                  90                  95
   Asp Lys Arg Ala Thr Glu Lys Lys Ile Asp Thr Thr Glu Ile Ala Asn
               100                 105                 110
   Leu Lys Thr Ala Val Glu Gly Leu Lys Asn Gln Asp Thr Gln Glu Asn
           115                 120                 125
   Phe Lys Ala Ala Ala Lys Asn Phe Lys Val Lys Val Ala Asn Ser Ile
       130                 135                 140
   Thr Asn Leu Lys Thr Val Ile Thr Glu Lys Asn Lys
   145                 150                 155
<210> 43
   <211> 154
   <212> PRT
   <213> 16SrV (Elm yellows group)
<400> 43
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Phe Trp Pro Phe Asn Pro Thr Ile Thr Glu
           35                  40                  45
   Lys Asp Leu Thr Thr Phe Lys Thr Asp Val Glu Lys Leu Val Glu Asp
       50                  55                  60
   Gly Lys Ala Thr Asp Val Lys Ala Asp Asn Asn Lys Ile Val Glu Ala
   65                  70                  75                  80
   Val Glu Asn Lys Ile Asn Ala Leu Lys Thr Gln Val Glu Gln Val Asn
                   85                  90                  95
   Lys Lys Arg Ala Asp Asn Lys Lys Ile Lys Thr Glu Lys Ile Asp Thr
               100                 105                 110
   Leu Lys Thr Glu Ala Val Lys Leu Lys Asp Lys Glu Thr Lys Val Ala
           115                 120                 125
   Phe Lys Glu Glu Ala Thr Lys Phe Lys Thr Lys Val Asp Thr Phe Val
       130                 135                 140
   Thr Glu Phe Lys Glu Glu Ile Lys Glu Lys
   145                 150
<210> 44
   <211> 153
   <212> PRT
   <213> 16SrV (Elm yellows group)
<400> 44
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Phe Trp Pro Phe Asn Pro Thr Ile Thr Glu
           35                  40                  45
   Lys Asp Leu Thr Thr Phe Lys Thr Asp Val Glu Lys Leu Val Glu Asp
       50                  55                  60
   Lys Lys Ala Thr Asp Val Glu Ala Asp Asn Asp Lys Ile Val Leu Asp
   65                  70                  75                  80
   Val Glu Lys Lys Ile Asn Ala Leu Lys Thr Gln Val Glu Glu Val Asn
                   85                  90                  95
   Lys Lys Arg Asp Asp Asp Lys Lys Ile Lys Thr Glu Lys Ile Asp Asn
               100                 105                 110
   Leu Lys Thr Glu Ala Gly Lys Leu Lys Gly Lys Lys Thr Lys Asp Thr
           115                 120                 125
   Phe Lys Asp Glu Ala Lys Lys Phe Lys Ala Ser Val Asp Thr Phe Val
       130                 135                 140
   Thr Glu Tyr Lys Thr Asp Ile Lys Lys
   145                 150
<210> 45
   <211> 154
   <212> PRT
   <213> 16SrV (Elm yellows group)
<400> 45
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Tyr Leu Ile
               20                  25                  30
   Ile Ala Tyr Phe Ala Ser Leu Tyr Pro Phe Asn Thr Thr Ile Thr Glu
           35                  40                  45
   Lys Asp Leu Thr Lys Phe Lys Thr Asp Val Glu Glu Leu Val Ala Asp
       50                  55                  60
   Gly Lys Glu Thr Asp Ala Lys Val Asp Asn Asn Lys Ile Val Glu Ala
   65                  70                  75                  80
   Val Glu Lys Lys Ile Asn Ala Leu Lys Glu Gln Val Glu Lys Val Asn
                   85                  90                  95
   Lys Lys Arg Ala Asp Asn Lys Lys Ile Lys Thr Glu Lys Val Asp Thr
               100                 105                 110
   Leu Lys Thr Glu Ala Gly Lys Leu Lys Asp Lys Glu Thr Lys Ile Ala
           115                 120                 125
   Phe Lys Glu Glu Ala Thr Lys Phe Lys Thr Lys Val Asn Thr Phe Val
       130                 135                 140
   Thr Glu Phe Lys Glu Glu Ile Lys Glu Lys
   145                 150
<210> 46
   <211> 149
   <212> PRT
   <213> 16SrV (Elm yellows group)
<400> 46
   Met Gln Gln Lys Glu Ser Phe Leu Lys Thr Lys Lys Gly Lys Ile Val
   1               5                   10                  15
   Phe Gly Ser Val Val Gly Val Leu Ala Leu Tyr Leu Leu Ile Ala Tyr
               20                  25                  30
   Phe Ala Ser Phe Trp Pro Phe Asn Thr Thr Ile Thr Glu Lys Asp Leu
           35                  40                  45
   Thr Thr Phe Lys Thr Asp Val Glu Lys Leu Val Ala Asp Glu Lys Ala
       50                  55                  60
   Thr Asp Val Glu Ala Asp Asn Asp Lys Ile Val Leu Asp Val Glu Lys
   65                  70                  75                  80
   Lys Ile Asn Ala Leu Lys Thr Gln Val Glu Glu Val Asn Lys Lys Arg
                   85                  90                  95
   Ala Asp Asp Lys Lys Ile Lys Pro Glu Lys Ile Asp Asn Leu Lys Ile
               100                 105                 110
   Glu Ala Asp Lys Leu Lys Gly Lys Lys Thr Lys Asp Thr Phe Lys Asp
           115                 120                 125
   Glu Ala Lys Lys Phe Lys Ala Ser Val Asp Lys Phe Val Thr Glu Tyr
       130                 135                 140
   Lys Thr Asp Ile Lys
   145
<210> 47
   <211> 152
   <212> PRT
   <213> 16SrV (Elm yellows group)
<400> 47
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Tyr Leu Ile
               20                  25                  30
   Ile Ala Tyr Ile Met Lys Trp Val Pro Phe Gly Ile Ser Ile Asn Asn
           35                  40                  45
   Thr Asp Leu Thr Thr Phe Lys Ala Asp Val Glu Lys Leu Val Ala Asp
       50                  55                  60
   Glu Lys Ala Thr Asp Val Lys Ala Asp Asn Asn Lys Ile Val Glu Ala
   65                  70                  75                  80
   Val Glu Asn Lys Ile Asn Ala Leu Lys Thr Gln Val Glu Gln Val Asn
                   85                  90                  95
   Glu Lys Arg Ala Asn Lys Lys Ile Lys Thr Glu Lys Ile Asp Thr Leu
               100                 105                 110
   Lys Thr Glu Ala Gly Lys Leu Lys Asp Lys Glu Thr Lys Thr Ala Phe
           115                 120                 125
   Lys Glu Glu Ala Thr Lys Phe Lys Thr Ser Val Asp Lys Phe Val Thr
       130                 135                 140
   Glu Phe Lys Glu Glu Ile Lys Glu
   145                 150
<210> 48
   <211> 152
   <212> PRT
   <213> 16SrV (Elm yellows group)
<400> 48
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Tyr Leu Ile
               20                  25                  30
   Val Ala Tyr Ile Ile Ser Met Trp Pro Phe Ala Ala Lys Ile Thr Glu
           35                  40                  45
   Lys Asp Leu Ala Thr Phe Lys Thr Asp Val Glu Lys Leu Val Ala Asp
       50                  55                  60
   Glu Lys Ala Gly Ser Lys Thr Glu Asn Asp Thr Thr Ala Lys Lys Val
   65                  70                  75                  80
   Glu Glu Arg Ile Glu Glu Val Lys Lys Leu Val Asp Lys Val Asn Glu
                   85                  90                  95
   Lys Arg Ala Ser Asp Lys Lys Ile Asp Thr Lys Lys Ile Glu Asp Leu
               100                 105                 110
   Lys Thr Glu Ala Gly Lys Leu Lys Asp Lys Glu Lys Glu Ala Phe Lys
           115                 120                 125
   Thr Val Ala Ala Glu Phe Lys Thr Lys Val Gly Ser Phe Val Thr Ser
       130                 135                 140
   Phe Lys Glu Asp Ile Lys Glu Lys
   145                 150
<210> 49
   <211> 154
   <212> PRT
   <213> 16SrV (Elm yellows group)
<400> 49
   Met Gln Gln Lys Glu Ser Phe Leu Lys Thr Lys Lys Gly Lys Ile Val
   1               5                   10                  15
   Val Gly Ser Val Val Gly Ser Ile Val Gly Leu Leu Ala Ile Leu Thr
               20                  25                  30
   Leu Cys Tyr Ile Met Ser Trp Phe Pro Phe Ser Pro Ser Ile Asn Glu
           35                  40                  45
   Thr Asp Val Asn Lys Phe Gly Asp Lys Val Lys Ala Leu Ile Val Asp
       50                  55                  60
   Gly Lys Val Lys Asp Ser Lys Asp Asp Asn Asn Lys Ile Phe Glu Asp
   65                  70                  75                  80
   Val Thr Lys Glu Ile Asp Thr Leu Lys Lys Lys Val Glu Ala Val Asn
                   85                  90                  95
   Asn Lys Arg Asn Thr Asp Lys Lys Ile Lys Thr Glu Glu Ile Asp Lys
               100                 105                 110
   Leu Lys Thr Lys Ala Glu Glu Leu Lys Asp Lys Glu Lys Thr Ala Phe
           115                 120                 125
   Lys Thr Ala Ala Val Lys Phe Lys Ala Glu Val Glu Asn Leu Leu Asp
       130                 135                 140
   Ser Leu Lys Ser Glu Ile Asn Ser Lys Lys
   145                 150
<210> 50
   <211> 152
   <212> PRT
   <213> 16SrV (Elm yellows group)
<400> 50
   Met Gln Gln Lys Glu Ser Phe Leu Lys Thr Lys Lys Gly Lys Ile Val
   1               5                   10                  15
   Phe Gly Ser Val Gly Ser Val Val Gly Val Leu Val Leu Tyr Leu Ile
               20                  25                  30
   Ile Ala Tyr Phe Val Ser Ile Trp Pro Phe Ser Pro Ser Ile Thr Asn
           35                  40                  45
   Lys Asp Leu Thr Thr Phe Lys Thr Asp Val Glu Lys Leu Val Thr Asp
       50                  55                  60
   Lys Glu Thr Asp Val Lys Leu Glu Asn Asp Lys Ile Ala Thr Glu Val
   65                  70                  75                  80
   Glu Lys Lys Ile Asp Asp Leu Lys Thr Gln Val Ile Lys Val Asn Lys
                   85                  90                  95
   Lys Arg Ala Asp Asn Lys Gln Ile Lys Thr Glu Lys Ile Asp Asn Leu
               100                 105                 110
   Lys Thr Glu Ala Gly Lys Leu Lys Gly Lys Glu Thr Lys Ala Ala Phe
           115                 120                 125
   Lys Asp Glu Ala Thr Lys Phe Lys Thr Asn Val Asp Thr Phe Ile Asn
       130                 135                 140
   Ser Phe Lys Glu Asp Ile Lys Glu
   145                 150
<210> 51
   <211> 154
   <212> PRT
   <213> 16SrV (Elm yellows group)
<400> 51
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Val Trp Pro Phe Asn Pro Thr Ile Thr Glu
           35                  40                  45
   Lys Asp Leu Thr Thr Phe Lys Thr Asp Val Glu Lys Leu Val Glu Asp
       50                  55                  60
   Gly Lys Ala Thr Asp Val Lys Ala Asp Asn Asn Lys Ile Val Glu Ala
   65                  70                  75                  80
   Val Glu Asn Lys Ile Asn Ala Leu Lys Thr Gln Val Glu Gln Val Asn
                   85                  90                  95
   Lys Lys Arg Ala Asp Asn Lys Lys Ile Lys Thr Glu Lys Ile Asp Thr
               100                 105                 110
   Leu Lys Thr Glu Ala Val Lys Leu Lys Asp Lys Glu Thr Lys Val Ala
           115                 120                 125
   Phe Lys Glu Glu Ala Thr Lys Phe Lys Thr Lys Val Asp Thr Phe Val
       130                 135                 140
   Thr Glu Phe Lys Glu Glu Ile Lys Glu Lys
   145                 150
<210> 52
   <211> 152
   <212> PRT
   <213> 16SrV (Elm yellows group)
<400> 52
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Thr Asn
           35                  40                  45
   Lys Asp Leu Glu Lys Phe Lys Thr Asp Val Glu Lys Leu Val Glu Asp
       50                  55                  60
   Glu Lys Ala Lys Asp Ser Lys Thr Glu Asn Asn Lys Ile Val Glu Thr
   65                  70                  75                  80
   Val Glu Asn Lys Ile Asn Ala Leu Lys Thr Gln Val Thr Thr Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Ala Lys Leu Lys Asp Lys Glu Thr Lys Thr Ala
           115                 120                 125
   Phe Asn Glu Glu Ala Thr Lys Phe Lys Ala Lys Val Asn Thr Phe Val
       130                 135                 140
   Thr Glu Phe Lys Glu Glu Ile Lys
   145                 150
<210> 53
   <211> 154
   <212> PRT
   <213> 16SrV (Elm yellows group)
<400> 53
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Tyr Leu Ile
               20                  25                  30
   Ile Ala Tyr Phe Ala Ser Leu Tyr Pro Phe Asn Thr Thr Ile Thr Glu
           35                  40                  45
   Lys Asp Leu Thr Thr Phe Lys Ile Asp Val Glu Glu Leu Val Ala Asp
       50                  55                  60
   Gly Lys Glu Thr Asp Ala Lys Ala Asp Asn Asn Lys Ile Val Glu Ala
   65                  70                  75                  80
   Val Glu Lys Lys Ile Asn Ala Leu Lys Thr Gln Val Glu Lys Val Asn
                   85                  90                  95
   Lys Lys Arg Ala Asp Asn Lys Lys Ile Lys Thr Glu Lys Val Asp Thr
               100                 105                 110
   Leu Lys Thr Glu Ala Gly Lys Leu Lys Asp Lys Glu Thr Lys Ile Ala
           115                 120                 125
   Phe Lys Glu Glu Ala Thr Lys Phe Lys Thr Lys Val Asn Thr Phe Val
       130                 135                 140
   Thr Glu Phe Lys Glu Glu Ile Lys Glu Lys
   145                 150
<210> 54
   <211> 154
   <212> PRT
   <213> 16SrV (Elm yellows group)
<400> 54
   Met Gln Gln Lys Glu Ser Phe Leu Lys Thr Lys Lys Gly Lys Ile Val
   1               5                   10                  15
   Val Gly Ser Val Gly Ser Val Gly Gly Val Leu Val Leu Tyr Leu Ile
               20                  25                  30
   Ile Ala Tyr Phe Ala Ser Leu Tyr Pro Phe Asn Thr Thr Ile Thr Glu
           35                  40                  45
   Lys Asp Leu Thr Lys Phe Lys Thr Asp Val Glu Glu Leu Val Ala Asp
       50                  55                  60
   Gly Lys Glu Thr Asp Ala Lys Val Asp Asn Asn Lys Ile Val Glu Ala
   65                  70                  75                  80
   Val Glu Lys Lys Ile Asn Ala Leu Lys Glu Gln Val Glu Lys Val Asn
                   85                  90                  95
   Lys Lys Arg Ala Asp Asn Lys Lys Ile Lys Thr Glu Lys Val Asp Thr
               100                 105                 110
   Leu Lys Thr Glu Ala Gly Lys Leu Lys Asp Lys Glu Thr Lys Ile Ala
           115                 120                 125
   Phe Lys Glu Glu Ala Thr Lys Phe Lys Thr Lys Val Asn Thr Phe Val
       130                 135                 140
   Thr Glu Phe Gln Glu Glu Ile Lys Glu Lys
   145                 150
<210> 55
   <211> 153
   <212> PRT
   <213> 16SrV (Elm yellows group)
<400> 55
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Phe Trp Pro Phe Ser Lys Lys Ile Thr Asn
           35                  40                  45
   Lys Asp Leu Glu Thr Phe Lys Thr Asp Val Glu Lys Leu Val Ala Asp
       50                  55                  60
   Glu Lys Ala Thr Asp Val Lys Thr Asp Asn Asn Lys Ile Val Glu Ala
   65                  70                  75                  80
   Val Glu Asn Lys Ile Asn Ala Leu Lys Ala Gln Val Glu Gln Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Val Lys Leu Lys Asp Lys Glu Lys Thr Ala Phe
           115                 120                 125
   Lys Glu Glu Ala Thr Lys Phe Lys Thr Lys Val Asn Thr Phe Val Thr
       130                 135                 140
   Glu Phe Lys Glu Glu Ile Lys Glu Lys
   145                 150
<210> 56
   <211> 153
   <212> PRT
   <213> 16SrV (Elm yellows group)
<400> 56
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
               20                  25                  30
   Ile Ala Tyr Val Met Lys Trp Ile Pro Phe Arg Val Lys Ile Thr Asn
           35                  40                  45
   Glu Asp Leu Glu Thr Phe Lys Thr Asn Val Glu Lys Leu Val Ala Asp
       50                  55                  60
   Asp Lys Ala Lys Glu Ala Lys Ala Asp Asn Asp Lys Ile Val Glu Glu
   65                  70                  75                  80
   Val Glu Asn Lys Ile Asn Ala Leu Lys Ala Gln Val Asp Gln Val Asn
                   85                  90                  95
   Lys Lys Arg Ala Ser Asp Lys Lys Ile Gly Thr Gln Lys Ile Glu Asp
               100                 105                 110
   Leu Lys Thr Glu Ala Gly Lys Leu Lys Asp Lys Glu Lys Ala Ala Phe
           115                 120                 125
   Lys Thr Ala Ala Thr Glu Phe Lys Thr Lys Val Gly Thr Phe Val Thr
       130                 135                 140
   Ser Phe Lys Glu Asp Ile Arg Glu Lys
   145                 150
<210> 57
   <211> 153
   <212> PRT
   <213> 16SrV (Elm yellows group)
<400> 57
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Thr Asp
           35                  40                  45
   Lys Asp Leu Glu Thr Phe Lys Thr Asp Val Glu Lys Leu Val Ala Asp
       50                  55                  60
   Glu Lys Ala Lys Asp Ala Lys Ala Asp Asn Asp Lys Ile Val Glu Glu
   65                  70                  75                  80
   Val Glu Lys Lys Ile Asn Ala Leu Lys Ala Gln Val Asp Lys Val Asn
                   85                  90                  95
   Lys Lys Arg Ala Ser Asp Lys Lys Ile Gly Thr Gln Lys Ile Glu Asp
               100                 105                 110
   Leu Lys Thr Glu Ala Gly Lys Leu Lys Asp Lys Asp Lys Ala Ala Phe
           115                 120                 125
   Lys Thr Ala Ala Thr Glu Phe Lys Thr Lys Val Gly Thr Phe Val Thr
       130                 135                 140
   Ser Phe Gly Glu Asp Ile Lys Glu Lys
   145                 150
<210> 58
   <211> 154
   <212> PRT
   <213> 16SrV (Elm yellows group)
<400> 58
   Met Gln Gln Lys Glu Ser Phe Leu Lys Thr Lys Lys Gly Lys Ile Val
   1               5                   10                  15
   Val Gly Ser Val Gly Ser Val Ile Gly Val Leu Val Leu Tyr Leu Ile
               20                  25                  30
   Ile Ala Tyr Ile Met Lys Trp Ile Pro Phe Arg Ile Ser Ile Asn Asp
           35                  40                  45
   Thr Asp Leu Thr Thr Phe Lys Thr Glu Val Glu Lys Leu Val Ala Asp
       50                  55                  60
   Asp Lys Ala Lys Asp Asp Lys Thr Glu Asn Asn Lys Ile Leu Glu Ser
   65                  70                  75                  80
   Val Glu Asn Lys Ile Asn Ala Leu Lys Lys Gln Val Glu Lys Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Val Lys Leu Lys Asp Lys Glu Thr Lys Ile Ala
           115                 120                 125
   Phe Asn Ala Glu Ala Thr Lys Phe Lys Thr Asn Val Asp Arg Phe Val
       130                 135                 140
   Thr Glu Phe Lys Glu Glu Ile Lys Glu Lys
   145                 150
<210> 59
   <211> 152
   <212> PRT
   <213> 16SrV (Elm yellows group)
<400> 59
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Asn Asp
           35                  40                  45
   Lys Asp Leu Glu Thr Phe Lys Thr Asp Val Glu Lys Leu Val Ala Asp
       50                  55                  60
   Asp Lys Ala Gly Ser Lys Thr Glu Asn Asn Thr Thr Ala Glu Lys Val
   65                  70                  75                  80
   Glu Glu Lys Ile Lys Glu Val Lys Lys Leu Val Asp Lys Val Asn Glu
                   85                  90                  95
   Lys Arg Ala Ser Asp Lys Lys Ile Asp Thr Lys Lys Ile Glu Asp Leu
               100                 105                 110
   Lys Thr Glu Ala Ala Lys Leu Lys Glu Lys Glu Lys Glu Ala Phe Lys
           115                 120                 125
   Thr Ala Ala Ala Glu Phe Lys Thr Lys Val Gly Ser Phe Val Thr Ser
       130                 135                 140
   Phe Lys Glu Asp Ile Lys Glu Lys
   145                 150
<210> 60
   <211> 152
   <212> PRT
   <213> 16SrV (Elm yellows group)
<400> 60
   Met Gln Gln Lys Glu Ser Phe Leu Lys Thr Lys Lys Gly Lys Ile Val
   1 5 10 15
   Val Gly Ser Val Gly Ser Val Val Gly Val Leu Val Leu Phe Leu Ile
   20 25 30
   Ile Ala Tyr Phe Ala Ser Ile Trp Pro Phe Ser Thr Lys Ile Thr Asp
   35 40 45
   Lys Asp Leu Glu Thr Phe Lys Thr Asp Val Glu Lys Leu Val Ala Asp
   50 55 60
   Asp Lys Ala Gly Ser Lys Thr Glu Asn Asn Thr Thr Ala Glu Lys Val
   65 70 75 80
   Glu Glu Lys Ile Lys Glu Val Lys Lys Leu Val Asp Lys Val Asn Glu
   85 90 95
   Lys Arg Ala Ser Asp Lys Lys Ile Asp Thr Lys Lys Ile Glu Asp Leu
   100 105 110
   Lys Thr Glu Ala Ala Lys Leu Lys Glu Lys Glu Lys Glu Ala Phe Lys
   115 120 125
   Thr Ala Ala Ala Glu Phe Lys Thr Lys Val Gly Ser Phe Val Thr Ser
   130 135 140
   Phe Lys Glu Asp Ile Lys Glu Lys
   145 150
<210> 61
   <211> 153
   <212> PRT
   <213> 16SrV (Elm yellows group)
<400> 61
   Met Gln Gln Lys Glu Ser Phe Leu Lys Thr Lys Lys Gly Lys Ile Val
   1 5 10 15
   Val Gly Ser Val Gly Ser Val Val Gly Val Leu Val Leu Phe Leu Ile
   20 25 30
   Ile Ala Tyr Phe Ala Ser Phe Trp Pro Phe Asn Pro Thr Ile Thr Glu
   35 40 45
   Lys Asp Leu Thr Thr Phe Lys Thr Asp Val Glu Lys Leu Val Ala Asp
   50 55 60
   Glu Lys Ala Thr Asp Val Glu Ala Asp Asn Asp Lys Ile Val Leu Asp
   65 70 75 80
   Val Glu Lys Lys Ile Asn Ala Leu Lys Thr Gln Val Glu Glu Val Asn
   85 90 95
   Lys Lys Arg Ala Asp Asp Lys Lys Ile Lys Thr Asp Lys Ile Asp Asn
   100 105 110
   Leu Lys Ile Glu Ala Asp Lys Leu Lys Gly Lys Lys Thr Lys Asp Thr
   115 120 125
   Phe Lys Asp Glu Ala Lys Lys Phe Lys Ala Ser Val Asp Thr Phe Val
   130 135 140
   Thr Gly Tyr Lys Thr Asp Ile Lys Glu
   145 150
<210> 62
   <211> 152
   <212> PRT
   <213> 16SrV (Elm yellows group)
<400> 62
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Ile Val Pro Phe Trp Pro Phe Ser Thr Lys Ile Thr Asn
           35                  40                  45
   Lys Asp Leu Glu Thr Phe Lys Lys Asp Val Glu Lys Leu Val Ala Asp
       50                  55                  60
   Glu Lys Ala Lys Asp Asp Lys Thr Asp Asn Asn Lys Ile Val Glu Ala
   65                  70                  75                  80
   Val Glu Asn Lys Ile Asn Ala Leu Lys Thr Gln Val Thr Thr Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Val Lys Leu Lys Asp Lys Glu Thr Lys Thr Ala
           115                 120                 125
   Phe Asn Glu Glu Ala Thr Lys Phe Lys Ala Lys Val Asn Thr Phe Val
       130                 135                 140
   Thr Glu Phe Lys Glu Glu Ile Lys
   145                 150
<210> 63
   <211> 152
   <212> PRT
   <213> 16SrV (Elm yellows group)
<220>
   <221> VARIANT
   <222> 25..130
<400> 63
   Met Gln Gln Lys Glu Ser Phe Leu Lys Thr Lys Lys Gly Lys Ile Val
   1               5                   10                  15
   Val Gly Ser Val Gly Ser Val Val Xaa Val Leu Val Leu Phe Leu Ile
               20                  25                  30
   Ile Ala Xaa Xaa Xaa Xaa Ile Trp Pro Phe Ser Thr Lys Ile Thr Asn
           35                  40                  45
   Lys Asp Leu Glu Lys Phe Lys Thr Asp Val Glu Lys Leu Val Glu Ser
       50                  55                  60
   Glu Gln Ala Lys Asp Ser Lys Thr Asp Asn Asn Lys Ile Xaa Glu Thr
   65                  70                  75                  80
   Val Glu Lys Lys Ile Asn Ala Leu Lys Thr Gln Val Thr Xaa Val Asn
                   85                  90                  95
   Glu Lys Arg Ala Asp Asn Lys Lys Ile Lys Thr Glu Lys Ile Asp Pro
               100                 105                 110
   Leu Lys Thr Glu Ala Ala Lys Leu Lys Asp Lys Xaa Thr Lys Thr Ala
           115                 120                 125
   Phe Xaa Glu Glu Ala Thr Lys Phe Lys Ala Lys Val Asn Thr Phe Val
       130                 135                 140
   Thr Glu Phe Lys Glu Glu Ile Lys
   145                 150
<210> 64
   <211> 153
   <212> PRT
   <213> 16SrV (Elm yellows group)
<400> 64
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Phe Trp Pro Phe Ser Lys Lys Ile Thr Asn
           35                  40                  45
   Lys Asp Leu Glu Thr Phe Lys Thr Asp Val Glu Lys Leu Val Ala Asp
       50                  55                  60
   Glu Lys Ala Thr Asp Val Lys Thr Asp Asn Asn Lys Ile Val Glu Ala
   65                  70                  75                  80
   Val Glu Asn Lys Ile Asn Ala Leu Lys Ala Gln Val Glu Gln Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Val Lys Leu Lys Asp Lys Glu Lys Thr Ala Phe
           115                 120                 125
   Lys Glu Glu Ala Thr Lys Phe Lys Thr Lys Val Asn Thr Phe Val Thr
       130                 135                 140
   Glu Phe Lys Glu Glu Ile Lys Glu Lys
   145                 150
<210> 65
   <211> 152
   <212> PRT
   <213> 16SrV (Elm yellows group)
<400> 65
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Thr Asn
           35                  40                  45
   Lys Asp Leu Glu Lys Phe Lys Thr Asp Val Glu Lys Leu Val Glu Asp
       50                  55                  60
   Glu Gln Ala Lys Asp Ser Lys Thr Asp Asn Asn Lys Ile Val Glu Thr
   65                  70                  75                  80
   Val Glu Lys Lys Ile Asn Asp Leu Lys Thr Gln Val Thr Thr Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Ala Lys Leu Lys Asp Lys Glu Thr Lys Thr Ala
           115                 120                 125
   Phe Asn Glu Glu Ala Thr Lys Phe Lys Ala Lys Val Asn Thr Phe Val
       130                 135                 140
   Thr Glu Phe Lys Glu Glu Ile Lys
   145                 150
<210> 66
   <211> 152
   <212> PRT
   <213> 16SrV (Elm yellows group)
<400> 66
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Thr Asn
           35                  40                  45
   Lys Asp Leu Glu Lys Phe Lys Thr Asp Val Glu Lys Leu Val Glu Asp
       50                  55                  60
   Glu Gln Ala Lys Asp Ser Lys Thr Asp Asn Asn Lys Ile Val Glu Thr
   65                  70                  75                  80
   Val Glu Lys Lys Ile Asn Ala Leu Lys Thr Gln Val Thr Thr Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Ala Lys Leu Lys Asp Lys Glu Thr Lys Thr Ala
           115                 120                 125
   Phe Asn Glu Glu Ala Thr Lys Phe Lys Ala Lys Val Asn Ala Phe Val
       130                 135                 140
   Thr Glu Phe Lys Glu Glu Ile Lys
   145                 150
<210> 67
   <211> 153
   <212> PRT
   <213> 16SrV (Elm yellows group)
<400> 67
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Leu Trp Pro Phe Ser Thr Lys Ile Thr Asn
           35                  40                  45
   Lys Asp Leu Glu Thr Phe Gln Lys Asp Val Glu Lys Leu Val Ala Asp
       50                  55                  60
   Glu Lys Ala Thr Asp Val Lys Thr Asp Asn Asn Lys Ile Val Glu Ala
   65                  70                  75                  80
   Val Glu Asn Lys Ile Asn Ala Leu Lys Thr Gln Val Asp Lys Val Asn
                   85                  90                  95
   Lys Lys Arg Ala Asp Asn Lys Lys Ile Lys Thr Glu Lys Ile Asp Thr
               100                 105                 110
   Leu Lys Thr Glu Ala Ala Lys Leu Lys Asp Lys Glu Lys Thr Ala Phe
           115                 120                 125
   Lys Glu Glu Ala Thr Lys Phe Lys Thr Asn Val Asp Lys Phe Val Thr
       130                 135                 140
   Glu Phe Lys Glu Glu Ile Lys Glu Lys
   145                 150
<210> 68
   <211> 152
   <212> PRT
   <213> 16SrV (Elm yellows group)
<400> 68
   Met Gln Gln Lys Glu Ser Phe Leu Lys Thr Lys Lys Gly Lys Ile Val
   1               5                   10                  15
   Val Gly Ser Val Gly Ser Val Ile Gly Val Leu Val Leu Tyr Leu Ile
               20                  25                  30
   Ile Ala Tyr Ile Met Lys Trp Ile Pro Phe Arg Ile Ser Ile Asn Asn
           35                  40                  45
   Thr Asp Leu Thr Thr Phe Lys Thr Glu Val Glu Lys Leu Val Ala Asp
       50                  55                  60
   Asp Lys Ala Thr Asp Val Lys Ala Asp Asn Asn Lys Ile Val Glu Ala
   65                  70                  75                  80
   Val Glu Glu Lys Ile Thr Ala Leu Lys Arg Gln Val Gly Lys Val Asn
                   85                  90                  95
   Glu Lys Arg Ala Asp Asp Lys Lys Ile Lys Thr Glu Lys Ile Asp Thr
               100                 105                 110
   Leu Lys Thr Asp Ala Gly Lys Leu Lys Asp Lys Glu Thr Lys Thr Ala
           115                 120                 125
   Phe Lys Glu Glu Ala Ala Lys Phe Lys Thr Ser Val Asn Lys Phe Val
       130                 135                 140
   Asn Glu Phe Lys Glu Glu Ile Asn
   145                 150
<210> 69
   <211> 152
   <212> PRT
   <213> 16SrV (Elm yellows group)
<400> 69
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Thr Asn
           35                  40                  45
   Lys Asp Leu Glu Lys Phe Lys Thr Asp Val Glu Lys Leu Val Glu Asp
       50                  55                  60
   Lys Gln Ala Lys Asp Ser Lys Thr Asp Asn Asn Lys Ile Val Glu Thr
   65                  70                  75                  80
   Val Glu Lys Lys Ile Asn Ala Leu Lys Thr Gln Val Thr Thr Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Ala Lys Leu Lys Asp Lys Glu Thr Lys Thr Ala
           115                 120                 125
   Phe Asn Glu Glu Ala Thr Lys Phe Lys Ala Lys Val Asn Ala Phe Val
       130                 135                 140
   Thr Glu Phe Lys Glu Glu Ile Lys
   145                 150
<210> 70
   <211> 152
   <212> PRT
   <213> 16SrV (Elm yellows group)
<400> 70
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Thr Asn
           35                  40                  45
   Lys Asp Leu Glu Lys Phe Lys Thr Asp Val Glu Lys Leu Val Glu Asp
       50                  55                  60
   Glu Lys Ala Lys Asp Ser Lys Thr Asp Asn Asn Lys Ile Val Glu Thr
   65                  70                  75                  80
   Val Glu Lys Lys Ile Asn Ala Leu Lys Thr Gln Val Thr Thr Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Ala Lys Leu Lys Asp Lys Glu Thr Lys Thr Ala
           115                 120                 125
   Phe Asn Glu Glu Ala Thr Lys Phe Lys Ala Lys Val Asn Thr Phe Val
       130                 135                 140
   Thr Glu Phe Lys Glu Glu Ile Lys
   145                 150
<210> 71
   <211> 152
   <212> PRT
   <213> 16SrV (Elm yellows group)
<400> 71
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Thr Asn
           35                  40                  45
   Lys Asp Leu Glu Lys Phe Lys Thr Asp Val Glu Lys Leu Val Glu Asp
       50                  55                  60
   Glu Gln Ala Lys Asp Ser Lys Thr Asp Asn Asn Lys Ile Val Glu Thr
   65                  70                  75                  80
   Val Glu Lys Lys Ile Asn Ala Leu Lys Thr Gln Val Thr Thr Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Ala Lys Leu Lys Asp Lys Glu Thr Lys Thr Ala
           115                 120                 125
   Phe Asn Glu Glu Ala Thr Lys Phe Lys Ala Lys Val Asn Thr Phe Val
       130                 135                 140
   Thr Glu Phe Lys Glu Glu Ile Lys
   145                 150
<210> 72
   <211> 154
   <212> PRT
   <213> 16SrV (Elm yellows group)
<400> 72
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Ile Trp Pro Phe Ser Thr Lys Ile Thr Asn
           35                  40                  45
   Lys Asp Leu Glu Thr Phe Lys Glu Asp Val Glu Lys Leu Val Ala Asp
       50                  55                  60
   Glu Lys Ala Thr Asn Val Lys Thr Asp Asn Asn Lys Ile Val Glu Glu
   65                  70                  75                  80
   Val Glu Lys Lys Ile Asn Ala Leu Lys Thr Gln Val Glu Lys Val Asn
                   85                  90                  95
   Lys Lys Arg Ala Asp Asn Lys Lys Ile Lys Thr Glu Lys Ile Asp Thr
               100                 105                 110
   Leu Lys Thr Glu Ala Val Lys Leu Lys Glu Lys Glu Thr Lys Asp Thr
           115                 120                 125
   Phe Lys Ser Glu Ala Ser Arg Phe Lys Ala Ser Val Asp Thr Phe Val
       130                 135                 140
   Thr Glu Phe Lys Glu Glu Ile Lys Glu Lys
   145                 150
<210> 73
   <211> 153
   <212> PRT
   <213> 16SrV (Elm yellows group)
<400> 73
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
               20                  25                  30
   Ile Ala Tyr Ile Met Lys Trp Ile Pro Phe Arg Glu Lys Ile Thr Asp
           35                  40                  45
   Lys Asp Leu Glu Thr Phe Lys Thr Asp Val Glu Lys Leu Val Ala Asp
       50                  55                  60
   Glu Lys Ala Met Asp Thr Lys Ala Asp Asn Asn Lys Ile Val Glu Ala
   65                  70                  75                  80
   Val Glu Lys Lys Ile Asn Ala Leu Lys Ala Gln Val Asp Lys Val Asn
                   85                  90                  95
   Glu Lys Arg Ala Ser Asp Lys Lys Ile Gly Thr Lys Lys Ile Glu Asp
               100                 105                 110
   Leu Lys Thr Glu Ala Gly Lys Leu Lys Asp Lys Glu Lys Glu Ala Phe
           115                 120                 125
   Lys Thr Val Ala Ala Glu Phe Lys Thr Lys Val Gly Asp Phe Val Thr
       130                 135                 140
   Ser Phe Lys Glu Asp Ile Lys Glu Lys
   145                 150
<210> 74
   <211> 153
   <212> PRT
   <213> 16SrV (Elm yellows group)
<400> 74
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
               20                  25                  30
   Ile Ala Tyr Ile Met Lys Trp Ile Pro Phe Arg Val Lys Ile Thr Asp
           35                  40                  45
   Lys Asp Leu Glu Thr Phe Lys Thr Asp Val Glu Lys Leu Val Ala Asp
       50                  55                  60
   Glu Lys Ala Thr Asp Thr Lys Ala Asp Asn Asn Lys Ile Val Glu Ala
   65                  70                  75                  80
   Val Glu Lys Lys Ile Asn Ala Leu Lys Ala Gln Val Asp Gln Val Asn
                   85                  90                  95
   Glu Lys Arg Ala Ser Asp Lys Lys Ile Gly Thr Lys Lys Ile Glu Asp
               100                 105                 110
   Leu Lys Thr Glu Ala Gly Lys Leu Lys Asp Lys Asp Lys Glu Ala Phe
           115                 120                 125
   Lys Thr Val Ala Ala Glu Phe Lys Thr Lys Val Gly Asp Phe Val Thr
       130                 135                 140
   Ser Phe Lys Glu Asp Ile Lys Glu Lys
   145                 150
<210> 75
   <211> 156
   <212> PRT
   <213> 16SrV (Elm yellows group)
<400> 75
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
               20                  25                  30
   Ile Ala Tyr Ile Met Lys Trp Ile Pro Phe Arg Glu Lys Ile Thr Pro
           35                  40                  45
   Glu Asp Ala Glu Ser Leu Lys Thr Lys Ile Glu Ser Leu Val Ala Asp
       50                  55                  60
   Thr Glu Ala Lys Thr Thr Ala Thr Asp Asn Asp Lys Ile Tyr Asp Lys
   65                  70                  75                  80
   Val Lys Lys Ala Ile Glu Ala Phe Glu Ser Lys Val Thr Ala Val Asn
                   85                  90                  95
   Glu Lys Arg Ala Thr Glu Lys Lys Ile Asp Thr Thr Glu Ile Thr Asn
               100                 105                 110
   Leu Lys Lys Glu Val Glu Asn Leu Lys Asn Gln Asp Thr Gln Asp Lys
           115                 120                 125
   Phe Lys Ala Ala Ala Lys Ile Phe Lys Asp Lys Val Ala Ile Ser Ile
       130                 135                 140
   Thr Asn Leu Lys Thr Val Ile Thr Glu Lys Asn Lys
   145                 150                 155
<210> 76
   <211> 156
   <212> PRT
   <213> 16SrV (Elm yellows group)
<400> 76
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
   20 25 30
   Ile Ala Tyr Ile Met Lys Trp Ile Pro Phe Arg Glu Lys Ile Thr Pro
   35 40 45
   Glu Asp Ala Glu Ser Leu Lys Thr Lys Ile Glu Ser Leu Val Ala Asn
   50 55 60
   Thr Glu Ala Lys Thr Thr Val Glu Glu Asn Asp Lys Ile Tyr Asp Lys
   65 70 75 80
   Val Lys Lys Ala Ile Glu Ala Phe Glu Ser Lys Val Lys Ala Val Asn
   85 90 95
   Glu Lys Arg Ala Thr Glu Lys Lys Ile Asp Thr Thr Glu Ile Thr Asn
   100 105 110
   Leu Lys Thr Ala Val Glu Gly Leu Lys Asn Lys Asn Thr Gln Glu Asn
   115 120 125
   Phe Lys Thr Ala Ala Lys Asp Phe Lys Asp Lys Val Ala Thr Ser Ile
   130 135 140
   Thr Asn Leu Lys Thr Val Ile Thr Glu Lys Asn Lys
   145 150 155
<210> 77
   <211> 153
   <212> PRT
   <213> 16SrV (Elm yellows group)
<400> 77
   Met Gln Gln Lys Glu Ser Phe Leu Lys Thr Lys Lys Gly Lys Ile Val
   1 5 10 15
   Val Gly Ser Val Ile Gly Ala Leu Val Leu Tyr Leu Ile Ile Ala Tyr
   20 25 30
   Ile Met Lys Trp Ile Pro Phe Arg Glu Lys Ile Thr Pro Glu Asp Ala
   35 40 45
   Glu Ser Leu Lys Thr Lys Ile Glu Ser Leu Val Ala Asn Thr Glu Ala
   50 55 60
   Lys Thr Thr Val Glu Glu Asn Asp Lys Ile Tyr Asp Lys Val Lys Lys
   65 70 75 80
   Ala Ile Glu Ala Phe Glu Ser Lys Val Lys Ala Val Asn Glu Lys Arg
   85 90 95
   Ala Thr Glu Lys Lys Ile Asp Thr Thr Glu Ile Thr Asn Leu Lys Thr
   100 105 110
   Ala Val Glu Gly Leu Lys Asn Lys Asn Thr Gln Glu Asn Phe Lys Thr
   115 120 125
   Ala Ala Lys Asp Phe Lys Asp Lys Val Ala Thr Ser Ile Thr Asn Leu
   130 135 140
   Lys Thr Val Ile Thr Glu Lys Asn Lys
   145 150
<210> 78
   <211> 153
   <212> PRT
   <213> 16SrV (Elm yellows group)
<400> 78
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
               20                  25                  30
   Ile Ala Tyr Ile Met Lys Trp Ile Pro Phe Arg Glu Lys Ile Thr Asp
           35                  40                  45
   Lys Asp Leu Glu Thr Phe Lys Thr Asp Val Glu Lys Leu Val Ala Asp
       50                  55                  60
   Glu Lys Ala Met Asp Thr Lys Ala Glu Asn Asn Lys Ile Val Glu Ala
   65                  70                  75                  80
   Val Glu Lys Lys Ile Asn Ala Leu Lys Ala Gln Val Asp Lys Val Asn
                   85                  90                  95
   Glu Lys Arg Ala Ser Asp Lys Lys Ile Gly Thr Lys Lys Ile Glu Asp
               100                 105                 110
   Leu Lys Thr Glu Ala Gly Lys Leu Lys Asp Lys Glu Lys Glu Ala Phe
           115                 120                 125
   Lys Thr Val Ala Ala Glu Phe Lys Thr Lys Val Gly Asp Phe Val Thr
       130                 135                 140
   Ser Phe Lys Glu Asp Ile Lys Glu Lys
   145                 150
<210> 79
   <211> 153
   <212> PRT
   <213> 16SrV (Elm yellows group)
<400> 79
   Met Gln Gln Lys Glu Ser Phe Leu Lys Thr Lys Lys Gly Lys Ile Val
   1               5                   10                  15
   Val Gly Ser Val Gly Ser Val Ile Gly Ala Leu Val Leu Tyr Leu Ile
               20                  25                  30
   Ile Ala Tyr Ile Met Lys Trp Ile Pro Phe Arg Glu Lys Ile Thr Asp
           35                  40                  45
   Lys Asp Leu Glu Thr Phe Lys Thr Asp Val Glu Lys Leu Val Ala Asp
       50                  55                  60
   Glu Lys Ala Met Asp Thr Lys Ala Glu Asn Asn Lys Ile Val Glu Ala
   65                  70                  75                  80
   Val Glu Lys Lys Ile Asn Ala Leu Lys Ala Gln Val Asp Lys Val Asn
                   85                  90                  95
   Glu Lys Arg Ala Ser Asp Lys Lys Ile Gly Thr Lys Lys Ile Glu Asp
               100                 105                 110
   Leu Lys Thr Glu Ala Gly Lys Leu Lys Asp Lys Glu Lys Glu Ala Phe
           115                 120                 125
   Lys Thr Val Ala Ala Glu Phe Lys Thr Lys Val Gly Asp Phe Val Thr
       130                 135                 140
   Ser Phe Lys Glu Asp Ile Lys Glu Lys
   145                 150
<210> 80
   <211> 154
   <212> PRT
   <213> 16SrV (Elm yellows group)
<400> 80
   Met Pro Gln Lys Glu Ser Phe Leu Lys Thr Lys Lys Gly Lys Ile Val
   1               5                   10                  15
   Val Gly Ser Val Gly Ser Val Ile Gly Val Leu Val Leu Tyr Leu Ile
               20                  25                  30
   Ile Ala Tyr Ile Met Lys Trp Ile Pro Phe Arg Ile Ser Ile Asn Asp
           35                  40                  45
   Thr Asp Leu Thr Thr Phe Lys Thr Glu Val Glu Lys Leu Val Ala Asp
       50                  55                  60
   Asp Lys Ala Lys Asp Asp Lys Thr Glu Asn Asn Lys Ile Leu Glu Ser
   65                  70                  75                  80
   Val Glu Asn Lys Ile Asn Ala Leu Lys Lys Gln Val Glu Lys Val Asn
                   85                  90                  95
   Glu Lys Arg Ala Asp Asn Lys Lys Ile Lys Thr Glu Lys Ile Asp Thr
               100                 105                 110
   Leu Lys Thr Glu Ala Val Lys Leu Lys Asp Lys Glu Thr Lys Ile Ala
           115                 120                 125
   Phe Asn Ala Glu Ala Thr Lys Phe Lys Thr Asn Val Asp Arg Phe Val
       130                 135                 140
   Thr Glu Phe Lys Glu Glu Ile Lys Glu Lys
   145                 150
<210> 81
   <211> 480
   <212> DNA
   <213> 16SrV (Elm yellows group)
<220>
   <221> allele
   <222> 115..431
<400> 81
   atgcmacmaa aagaaagttt tttaaaaact aaaaaaggya aaatagttkt tggttcagtt      60
   gttggttcar ttrktggkky wttagyawta twtttamyav ttksttakdt krbrncanwk     120
   krdthccatt yvvkvhawma attamtvmdr magatkyarm mavwttwvrr rhwrawrtwr     180
   aadmattart arharryrvh vaarhaahrr vwdhkrhadh wganaatrac amaayakhwd     240
   hmdmwgtrrm aramrvaaty rmhrmwktdr aanvrmwagt rgrnhvmrgt taatramaaa     300
   agarmtdmmr amaaamaaat trrhmcwvmh raartarmmv mhttaaaaah armmgytgnm     360
   rrwttaaaar rhmaaraham wvmarhhrmw tttaadrvwg hagcwrndrd wttcaaarhm     420
   rrmgtyrvwr nwtymdtarm wrrwtwmvra dmagwmatta cagaaarwad yaaawaataa     480
<210> 82
   <211> 152
   <212> PRT
   <213> 16SrV (Elm yellows group)
<400> 82
   Met Gln Gln Lys Glu Ser Phe Leu Lys Thr Lys Lys Gly Lys Ile Val
   1               5                   10                  15
   Val Gly Ser Val Gly Ser Val Val Gly Val Leu Val Leu Phe Leu Ile
               20                  25                  30
   Ile Ala Tyr Phe Ala Ser Trp Trp Phe Ser Thr Lys Ile Thr Asn Lys
           35                  40                  45
   Asp Leu Glu Thr Phe Lys Thr Asp Val Glu Lys Leu Val Ala Asp Glu
       50                  55                  60
   Lys Ala Lys Asp Val Lys Thr Asp Asn Asn Lys Ile Val Glu Ala Val
   65                  70                  75                  80
   Glu Lys Lys Ile Asn Ala Leu Lys Thr Gln Val Glu Lys Val Asn Glu
                   85                  90                  95
   Lys Arg Ala Asn Lys Lys Ile Lys Thr Glu Lys Ile Asp Thr Leu Lys
               100                 105                 110
   Thr Glu Ala Gly Lys Leu Lys Asp Lys Glu Thr Lys Thr Ala Phe Lys
           115                 120                 125
   Glu Glu Ala Thr Lys Phe Lys Thr Lys Val Asn Thr Phe Val Thr Glu
       130                 135                 140
   Phe Lys Glu Glu Ile Lys Glu Lys
   145                 150
<210> 83
   <211> 21
   <212> DNA
   <213> 16SrV (Elm yellows group)
<400> 83
   tagagagaat tttaggccac g 21
<210> 84
   <211> 22
   <212> DNA
   <213> 16SrV (Elm yellows group)
<400> 84
   atagaaaata acgataaagc ag 22
<210> 85
   <211> 22
   <212> DNA
   <213> 16SrV (Elm yellows group)
<400> 85
   aataatgaag aacaattacc tg 22
<210> 86
   <211> 23
   <212> DNA
   <213> 16SrV (Elm yellows group)
<400> 86
   tcaagacctt ttaaaccaca ccc 23

## Description

### Technical Field

This invention relates to phytoplasma strains causing flavescence dorée of grapevine as well as to phylogenetically similar phytoplasmas, and to use of such sequences.

### Background of the invention

Phytoplasmoses are severe diseases of grapevine caused by unicellular microorganisms, so called phytoplasmas, belonging to the class of Mollicutes and without cell wall. They can be grown with extreme difficulty on artificial substrates (Contaldo et al., 2012, [10]), because they are highly evolved endoparasites which, in order to survive, necessitate several metabolites that are present in the phloem of plants. Phytoplasmas in fact live at the level of phloem sieve tubes where they multiply uninterruptedly; in nature they are conveyed by insect vectors such as leafhoppers and psyllids.

Through examination of molecular features a division of phytoplasmas in different phylogenetic groups has been established (ribosomal groups 16Sr), each of which includes a high number of sub-groups (Lee et al., 1998, [19]; Seemüller et al., 1998, [28]). Recent taxonomy classifies phytoplasmas into the new genus "Candidatus (Ca.) Phytoplasma" (IRPCM, 2004), which presently contains over 20 different species.

The diseases that phytoplasmas cause to the grapevine are commonly referred to under the name of "grapevine yellows". They include severe infectious forms that were discovered around the middle Fifties and are present in many grapevine areas all over the world, but particularly in Europe. There are two diseases that, over the years, have acquired great importance because of their dangerousness and harmfulness : black wood, whose etiological agent belongs to the species "*Ca.* Phytoplasma *solani*"*,* and flavescence dorée (FD), caused by phytoplasmas of the ribosomal group 16SrV, which have not been classified into an officially described species yet.

Black wood is considered a not very dangerous disease, with a definite behavior of endemic kind because of its slow and gradual diffusion. FD, instead, has shown as form its first appearance, which took place in France around the year 1950 (Caudwell, 1957, [9]), a very serious epidemic character and according to the regulations provided by the European Union it is included among quarantine diseases (Council Directive 2000/29/EU of 8 May 2000, Annex II, A2). Nevertheless, this disease has been spreading in virulent form at first in Corsica and Northern Italy as from the Seventies (Belli et al., 1973, [6]), and the in Middle Italy and Southern Italy. Nowadays it is present in France, Italy, Spain (Batlle et al., 1997, [5]), Portugal (De Sousa et al., 2009, [11]), Austria (Reisenzein et al., 2011, [25]), Switzerland (Gugerli et al., 2006, [15]), Slovenia (Maixner, 2006, [21]]), Croatia (Šeruga Music et al., 2011, [29]) and Serbia (Duduk et al., 2003, [12], Kuzmanovič̌̌̌ et al., 2008, [18]). Its diffusion is worrying especially because there is a constant advance of epidemics over the years, which apparently cannot be stopped.

The symptoms of this disease are evident on leaves, shoots and grape bunches (Angelini et al., 2010, [3]). Leaves show very evident color alterations with yellow or reddish hues on white and black varieties, respectively; furthermore they show blistering and have a papyraceous texture and the margins are turned towards the lower leaf blade in many varieties. Shoot are willowy and rubbery until late season and have reduced and irregular lignification, sometimes with pustules at the basis of the shoot. Flower heads and clusters present on sick shoots dry up before they reach maturation, thus causing serious damages to vine growers. There are two kinds of damages: 1) decrease in the quantity and quality of productions owing to the loss of clusters and lack of maturation of the grapevine; 2) gradual weakening of the plants, caused by the duration of symptoms over several years, which brings about a progressive deterioration of the planting. The more severe the symptoms are and the younger the affected plants are, the more rapid and intense the general decline of grapevines is.

The FD is transmitted from grapevine to grapevine specifically by *Scaphoideus titanus* Ball., a leafhopper of America origin which passes its entire life cycle on *Vitis* spp. and spreads the FD in an epidemic manner (Schvester et al., 1961, [26]; Mori et al., 2002, [24]). This insect transmits the FD phytoplasma in a very effective way while it feeds, by migrating from sick grapevines to healthy grapevines. Another leafhopper vector of the FD phytoplasma has recently been found, namely *Dictyophara europaea* Linnaeus, capable of transmitting the phytoplasma from infected clematis to the grapevine, even if it seems that such transmissions occurs rarely (Filippin et al., 2009, [13]). The presence of the FD phytoplasma has been further identified in trees of *Ailanthus altissima* (Filippin et al., 2010, [14]), common alder (Maixner et al., 1999, [20]; Arnaud et al., 2007, [4]) and in leafhopper of the species *Orientus ishidae* (Mehle et al., 2010, [23]), although the possible role of said species in FD epidemics is still to be cleared up. The transmission of the etiological agent is possible also by grafting of grapevine material, even if it has been proved that the risk of obtaining sick vine varieties is very low.

The fight against FD is based mainly on insecticidal interventions against the vector. The success of control depends on the strategies adopted in the entire area, so as to avoid new migrations of the insect from vineyards that are not properly defended. Community phytosanitary regulations (Directive 2000/29/EU mentioned above) compels member states to adopt measures useful for avoiding diffusion of the disease and of its vector within the Community. In grapevine areas already struck by the disease and affected by the presence of the vector, the fight is often compulsory and the number of insecticidal treatments to be carried out depends on national laws or regional regulations.

Therefore, punctual and real-time monitoring of the sanitary status of vineyards, performed by means of reliable and sizable analysis, becomes essential for the success of FD containment and fight measures. Late recognition can make treatments unsuccessful and cause the need to proceed to eradicating the crops, with even greater economic damages.

### Prior Art

Most of the diagnostic techniques used for other pathogenic microorganisms have proved inadequate for the diagnosis of FD phytoplasmas. Indeed, neither visual observation of symptoms nor transmission by grafting nor electronic microscopy are methods useful for carrying out routine diagnoses of this disease. *In vitro* culture, too, is not a valid method, due to the severe difficulty in culturing phytoplasmas outside their hosts, as mentioned above.

At present, the most used for FD diagnosis is molecular diagnostics based on DNA extraction and PCR (Polymerase Chain Reaction) assay. As phytoplasmas are scarcely concentrated in grapevine tissues, it is necessary to effect two subsequent conventional PCR reactions (nested PCR) or to use real-time PCR, which is more sensitive than conventional PCR.

A PCR-based diagnostic method is described in WO 2012/017271. This document describes nucleotide sequences coding for the *rpl14* gene of phytoplasmas belonging to the sub-groups 16SrV-C and 16SrV-D of the group 16SrV, to which, as has been demonstrated, all the strains of phytoplasmas causing FD belong, sequences coding for the corresponding protein L14, as well as methods and diagnostic kits using such sequences.

Molecular diagnostic methods like the aforementioned one are very reliable, accurate and very sensitive, but they require highly specialized personnel as well as quite long times and are therefore relatively expensive.

For handling this disease it would therefore be interesting to establish also a method that is more rapid and inexpensive and more easily applicable.

One of the methods that better suits the analysis of several samples and the monitoring of quarantine diseases is the serological assay, which is based on the reaction of antibodies that recognize proteins specific for the pathogenic agent, so called antigens. In particular the ELISA (Enzyme-Linked ImmunoSorbent Assay) assay is used for routine diagnosis of a large number of pathogens such as viruses, because it is easy to apply, takes short times, does not require highly specialized personnel and has costs lower than those of molecular biology assays. On the other hand, the serological assay is less sensitive than the molecular one.

In the past, some serological assays were developed (Boudon-Padieu et al., 1989, [8]; Seddas et al., 1996, [27]), that have proved to be valid for analysis on model plants or on vectors, but are not appropriate for diagnosis on grapevine just because of their too low sensitivity. Therefore such anti-serums, though available on the market (sold by SEDIAG, France), are not normally used.

The main problem in developing a serological assay for phytoplasmas is the difficulty in identifying phytoplasma proteins suitable for acting as antigen. These should be membrane proteins that have an outer portion for allowing action by antibodies, have a high concentration and are specific to the FD phytoplasma. The most suitable proteins for this purpose are the so-called immunodominant membrane proteins (IDP, ImmunoDominant membrane Protein), which, in phytoplasmas, represent the majority of the proteins of the cell membrane, in direct contact with the outer environment (Kakizawa et al., 2006, [16]). Among these there are IMP proteins, that are coded by the *imp* gene that the inventors have identified in strains of FD and of similar phytoplasmas in the ribosomal group 16SrV.

In this connection it is to be noted that the *rpl14* gene whose sequences are described in WO 2012/017271 can hardly be used for making serological assays: indeed, the protein coded by said gene is located inside the phytoplasma and therefore is clearly unsuitable for acting as antigen.

### Summary of the invention

The present invention provides, according to a first aspect, a nucleotide sequence SEQ.ID.NO.81, and its complementary sequence coding for the *imp* gene of phytoplasmas belonging to the ribosomal group 16SrV.

According to an advantageous feature of the invention, there are also provided nucleotide sequences, from SEQ.ID.NO.1 through SEQ.ID.NO.40, and sequences complementary thereto, of a genomic region of such phytoplasmas that extends on both sides of the *imp* gene and includes, besides this gene, terminal parts of the two genes flanking the *imp* gene, namely the *dnaD* gene, coding for a protein involved in the start of chromosomal replication, and the *pyrG* gene, coding for cytidine triphosphate synthetase.

According to another aspect of the invention, an amino acid sequence SEQ.ID.NO.82 is provided coding for immunodominant membrane proteins of phytoplasmas belonging to the ribosomal group 16SrV.

Advantageously, the amino acid sequence according to the invention summarizes the sequences from SEQ.ID.NO.41 through SEQ.ID.NO.80.

Disclosed is also at least a pair of nucleotide sequences that comprises a fragment of at least one of the nucleotide sequences of the *dnaD* gene or of the complementary sequences, and a fragment of the least one of the nucleotide sequence of the *pyrG* gene or of the complementary sequences, said sequences being one of the sequences SEQ.ID.NO.1 through SEQ.ID.NO.40 or the sequences complementary to said one of the sequences SEQ.ID.NO.1 through SEQ.ID.NO.40 respectively, the sequences of the pair being capable of being used as primers for amplification or for subsequent characterization at least of the *imp* gene of phytoplasmas belonging to the ribosomal group 16SrV.

Preferably, the sequences of said pair have a length of 18-25 nt, of which at least 15 nt belonging to at least one of the nucleotide sequences of the *dnaD* gene or of the complementary sequences and respectively to at least one of the nucleotide sequences of the *pyrG* gene or of the complementary sequences.

As is known to experts, indeed, the first object of a primer for amplification of a sequence is specificity. As a general rule, the longer the primer is, the higher the specificity is. There are mathematical equations that serve to determine the probability that a sequence perfectly complementary to a series of nucleotides is found by chance inside a DNA sequence formed by casual nucleotides. Such equations foresee that, in a genome consisting of 3x10⁹ nucleotides, a fragment of 15 nucleotides is represented only once. Considering the discrepancy between theoretical genomes and actual genomes, which are not casual sequences of nucleotides, it is advisable to use nucleotides that are longer than the statistical result. The priming region complementary to the DNA to be amplified should therefore be 18-25 nt long, but primers with a length from 15 to 27 nt have also been successfully used (Sambrok et al, 2001, [30]; Mitsuhashi, 1996, [31]).

According to the document Filippin Luisa: "Scientific Report of Luisa Filippin on the STSM (ref. code: COST-STSM-FA0807-05285)", 2010, pages 1-4, XP055133519, available on the Internet: URL:http://www.costphytoplasma.ipwgnet.org/PDF files/STSM/ Luisa Filippin Report for COST Website.pdf, primers have been designed and tested for amplifying by means of nested PCR the *imp* gene in isolates of the group 16SrV. A region comprising the *imp* gene and part of the flanking genes on both sides have been amplified. The best results have been obtained with two pairs of primers amplifying about 800 - 1000 nt. The document does not provide any indications as to a nucleotide sequence coding for the terminal part of the *dnaD* gene, the initial part of the *pyrG* gene or the *imp* gene or as to the corresponding amino acid sequence coding for IMP proteins, and therefore it does not provide suggestions for using as primers fragment of the sequences SEQ.ID.NO.1 through SEQ.ID.NO.40.

Document DA ROLD G ET AL: "The imp gene in Flavescence doree and related phytoplasmas for grapevine, clematis and alder", 18TH INTERNATIONAL CONGRESS OF INTERNATIONAL ORGANISATION OF MYCOPLASMOLOGY (IOM CONGRESS 2010), SIENA, ITALY, JULY 10-16-2010, 10 July 2010 (2010-07-10), XP009179542, and document G. Da Rold, L. Filippin, M. Borgo and E. Angelini: "Characterization of the imp gene in flavescence dorée and related phytoiplasmas" page 100, available on the Internet at http://www.bulletinofinsectology.org/pdfarticles/vol64-2011-S049-S050filippin.pdf, relate to the imp gene, dnaD-imp-pyrG genomic region and primers to amplify this region without however specifying any sequence.

Although this is obvious for the person skilled in the art, it has to be noted that, given a nucleotide sequence, the definition of its complementary sequence is immediate: therefore, the sequences complementary to the sequences SEQ.ID.NO.1 through SEQ.ID.NO.40 and SEQ.ID.NO.81 or to their fragments are not reported in the description.

The invention further relates to the use of such sequences or their fragment for the diagnosis of flavescence dorée and other pathologies caused by phytoplasmas belonging to the ribosomal group 16SrV.

Diagnosis can be effected by means of molecular methods or serological methods.

As mentioned, immunodominant membrane proteins constitute the majority of cell membrane proteins in phytoplasmas, in direct contact with the outer environment, and therefore they are well suited for acting as antigen in a serological diagnostic method. The invention therefore solves the problems set forth above relating to molecular diagnostic methods.

In any case, the primers provided can be used also in a molecular method, which can be an alternative to the methods that have already been described in specialized literature and are presently used, but can also provide genetic information that cannot be obtained with the currently known methods. Indeed, this method, being based on a very polymorphic gene, as will be described hereinafter, is useful for very finely characterizing, at molecular level, strains other than those of FD and similar phytoplasmas. Therefore it can be used for a "traceability" of infection sources, after appropriate sequencing and characterization of the PCR products. This possibility remarkably increase the versatility of the invention.

A further contribution to the high versatility of the invention is provided by the fact that the sequences provided can be used for identifying non only FD-specific phytoplasmas, belonging to the ribosomal sub-groups 16SrV-C and 16SrV-D, but also other phytoplasmas of the group 16SrV, in particular of the sub-groups 16SrV-C, 16SrV-D and 16SrV-E, which are more commonly present in other crops or plants (for instance hemp, alder, bramble, etc.) and some of which can also be present on grapevine.

### Brief Description of the Drawings

These and other features and advantages of the present invention will be clear from the following detailed description given by way of non-limiting example with reference to the attached drawing showing the genomic region *dnaD-imp-pyrG* of the strains of the phytoplasmas of the ribosomal group 16SrV and the position of primers that can be used for the diagnosis of pathologies caused by said phytoplasmas.

### Detailed Description of the Invention

As is known to experts, there are several strains of phytoplasmas causing FD, which are distinguished at the molecular level but also by their geographical distribution. In particular, it has been shown that all the strains of phytoplasmas causing FD belong to the ribosomal sub-groups 16SrV-C and 16SrV-D. The strains of the type 16SrV-D are the most widespread in European vineyards, seems to be mutually identical and probably have clonal origin; the reference strains are FD-D (Italian) and FD92 (French), which are identical to each other (Angelini et al., 2001, [1]; Arnaud et al., 2007, [4]). The strains of the type 16SrV-C, instead, differ with respect to the geographical area, and are usually different from country to country. There are strains that have been found only in Italy (some of them being typical of north-eastern vineyards, others being typical of the north-western regions of Italy, some others being typical of central Italy) (Martini et al., 2002, [22]; Botti et al., 2007, [7]; Filippin et al., 2009, [13]); strains that have been found only in France (FD70, FD2000, FDCAM) (Arnaud et al., 2007, [4]); other strains that have been found only in vineyards in Serbia, or Slovenia or Austria (Angelini, unpublished data).

In addition, many of the strains of phytoplasmas causing FD on grapevine have been found also on clematis, alder and ailanthus, at least in Italy, France and in the Balkans, countries/geographical areas where this disease has been most studied. Furthermore, other phytoplasmas, these too belonging to the phylogenetic subgroups 16SrV-C and D, that potentially might cause FD in vineyards if transmitted to grapevine have also been found on these spontaneous plants (Angelini et al., 2004, [2]; Arnaud et al., 2007, [4]; Filippin et al., 2010, [14]; Maixner et al., 1999, [20]).

There are also other phytoplasmas similar to those of FD, these too belonging to the phylogenetic group 16SrV, that have however been found on other plants where they cause different diseases, as will appear from the description hereinafter.

The nucleotide sequences of the invention allow to characterize the *imp* gene coding for IMP proteins of strains of phytoplasmas belonging to the ribosomal group 16SrV and comprising both strains specifically responsible for flavescence dorée (belonging to the sub-groups 16SrV-C and 16SrV-D) and phylogenetically similar strains belonging to the sub-groups16SrV-C, 16SrV-D and 16SrV-E.

In this way diagnostic assays can be provided that allow to identify also other pathogens that may be present on grapevine, though less frequently and with less devastating effects than the pathogens causing FD, and other diseases.

Table 1 below shows the strains of the group 16SrV characterized so far at the *imp* gene level.

**Table 1**

| No. | Strain code | Host | Geographical origin | Classification |
|---|---|---|---|---|
| 1 | FD-D | grapevine | Italy | 16SrV-D |
| 2 | FD92 | grapevine | France | 16SrV-D |
| 3 | FD-C | grapevine | Italy | 16SrV-C |
| 4 | FD70 | grapevine | France | 16SrV-C |
| 5 | FD2000 | grapevine | France | 16SrV-C |
| 6 | PGY-A | grapevine | Germany | 16SrV-C |
| 7 | PGY-C | grapevine | Germany | 16SrV-C |
| 8 | ALY | alder | Italy | 16SrV-C |
| 9 | HDI | hemp | USA | 16SrV-C |
| 10 | RUS | *Rubus* spp | Italy | 16SrV-E |
| 11 | Vv-AL-23 | grapevine | Italy, AL | 16SrV-C |
| 12 | Vv-TN-364 | grapevine | Italy, TN | 16SrV-C |
| 13 | Vv-SI-257 | grapevine | Italy, SI | 16SrV-C |
| 14 | Vv-AP-2 | grapevine | Italy, AP | 16SrV-C |
| 15 | Vv-PG-204 | grapevine | Italy, PG | 16SrV-C |
| 16 | Vv-Austria-816 | grapevine | Austria | 16SrV-C |
| 17 | Vv-Slovenia-1 | grapevine | Slovenia | 16SrV-C |
| 18 | Vv-Serbia-29 | grapevine | Serbia | 16SrV-C |
| 19 | Ag-TV-14 | alder | Italy, TV | 16SrV-C |
| 20 | Ag-TV-31 | alder | Italy, TV | 16SrV-C |
| 21 | Ag-TV-25 | alder | Italy, TV | 16SrV-C |
| 22 | Cv-AL-31 | clematis | Italy, AL | 16SrV-C |
| 23 | Cv-TV-33 | clematis | Italy, TV | 16SrV-C |
| 24 | Cv-SI-118 | clematis | Italy, SI | 16SrV-C |
| 25 | Cv-UD-102 | clematis | Italy, UD | 16SrV-C |
| 26 | Cv-UD-147 | clematis | Italy, UD | 16SrV-C |
| 27 | Cv-Slovenia-169 | clematis | Slovenia | 16SrV-C |
| 28 | Cv-Macedonia-99 | clematis | Macedonia | 16SrV-C |
| 29 | Aa-UD-65 | ailanthus | Italy, UD | 16SrV-C |
| 30 | Aa-UD-101 | ailanthus | Italy, UD | 16SrV-C |
| 31 | Aa-TV-54 | ailanthus | Italy, TV | 16SrV-C |
| 32 | Aa-TV-76 | ailanthus | Italy, TV | 16SrV-C |
| 33 | Aa-TV-21 | ailanthus | Italy, TV | 16SrV-C |
| 34 | Aa-TV-11 | ailanthus | Italy, TV | 16SrV-C |
| 35 | Aa-TV-46 | ailanthus | Italy, TV | 16SrV-C |
| 36 | Aa-TV-44 | ailanthus | Italy, TV | 16SrV-C |
| 37 | Aa-TV-107 | ailanthus | Italy, TV | 16SrV-C |
| 38 | Aa-VI-94 | ailanthus | Italy, VI | 16SrV-C |
| 39 | St-TV-1002 | *Scaphoideus titanus* | Italy, TV | 16SrV-C |
| 40 | St-Serbia-202 | *Scaphoideus titanus* | Serbia | 16SrV-C |

Interpretation of the acronyms appearing in column 2 is reported below:
- FD:: strains of the phytoplasma causing FD (flavescence dorée);
- PGY:: strains causing Palatinate Grapevine Yellows;
- ALY:: reference strain of the phytoplasma causing Alder Yellows;
- HDI:: reference strain of the phytoplasma causing Hemp Dogbane;
- RUS:: reference strain of the phytoplasma causing Rubus Stunt;
- Vv:: strains of the FD phytoplasma found by the inventors on *Vitis vinifera*;
- Ag:: strains similar to the FD phytoplasma and found by the inventors on *Alnus glutinosa* (black alder);
- Cv:: strains similar to the FD phytoplasma and found by the inventors on *Clematis vitalba* (clematis);
- Aa:: strains similar to the FD phytoplasma and found by the inventors on *Ailanthus altissima* (ailanthus);
- St:: strains of FD phytoplasma found by the inventors on *Scaphoideus titanus* (insect vector of FD).

In FD strains the acronym is followed by the indication of the year in which they have been discovered or by the indication of the phylogenetic group to which they belong; in PGY strains the acronym is followed by a letter allocated to the strain by its discoverers; in Vv, Ag, Cv, Aa, St strains the acronym is followed by the abbreviation of the province (for strains found in Italy) or by the country name (for strains found outside Italy) and by a number.

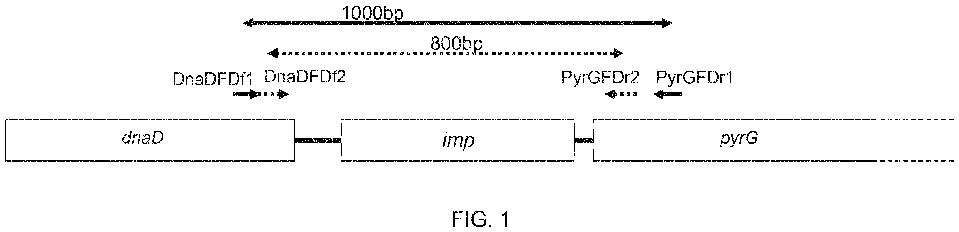

Annex 1 shows the alignment of the nucleotide sequences of the *dnaD-imp-pyrG* gene region of each of the above-listed strains, which region comprises, besides the *imp* gene, at least one terminal part of the *dnaD* gene coding for a protein involved in the start of chromosomal replication, and at least one terminal part of the *pyrG* gene, coding for a protein known as cytidine triphosphate synthetase, besides the intergenic gaps. Said gene region is graphically illustrated in Fig. 1.

It is noted that the *pyrG* gene is antisense relative to the other two, and therefore the part shown in Annex 1 is the terminal part of the gene itself.

The nucleotide sequences are identified in Annex 1, besides being identified by means of the strain code, are identified also by a sequence number (SEQ.ID.NO.1, SEQ.ID.NO.2 ... SEQ.ID.NO.40) corresponding to the number provided in column 1 of the table. The end of the *dnaD* gene (the block written in underlined bold italics), the beginning and the end of the *imp* gene (the blocks written in bold italics with double underline), and the end of the *pyrG* gene (the block written in underline bold italics) are shown in the Appendix. A comparison of the sequences SEQ.ID.NO.1 - SEQ.ID.NO.40 clearly shows the high polymorphism of the *imp* gene. The asterisk in the final line of each nucleotide block of the sequences indicates, in a conventional way, that a nucleotide is maintained in a certain position in all the sequences, whereas the absence of symbols indicates maintaining of less than 100%.

It is pointed out that in this description, when speaking of nucleotide sequence, one always means both the sequence reported in the text and the complementary sequence (which is not reported for the sake of simplicity), even if this is not explicitly stated.

The amino acid sequences of Annex 2 correspond to the nucleotide sequences of Annex 1. Also the sequences of Annex 2, besides being identified by means of the strain code, are identified also by a sequence number corresponding to the number provide in column 1 of Table 1 augmented by 40 (SEQ.ID.NO.41 - SEQ.ID.NO.80). The amino acid sequences SEQ.ID.NO.41 - SEQ.ID.NO.80 can be obtained from the nucleotide sequences SEQ.ID.NO.1 - SEQ.ID.NO.40 in a way known to experts in the field.

In the final line of each block of amino acids, the symbols "*", ":" and "." indicate respectively, in a conventional way, the fact that an amino acid is maintained in a certain position in all the sequences, a high similarity (> 75%) and a middle similarity (50% - 75%) among the sequences, whereas the absence of symbols indicate a similarity lower than 50%.

The sequences SEQ.ID.NO.1 through SEQ.ID.NO.40 and SEQ.ID.NO.41 through SEQ.ID.NO.80 are expressed according to the IUPAC (International Union of Pure and Applied Chemistry) nomenclature. For clarity, Tables 2 and 3 herein provide the abbreviations used by IUPAC for representing nucleotides (or bases) and amino acids.

**Table 2**

| IUPAC Code | Nucleotide (Base) |
|---|---|
| A | Adenine |
| C | Cytosine |
| G | Guanine |
| T (or U) | Thymine (or Uracil) |
| R | A or G |
| Y | C or T |
| S | G or C |
| W | A or T |
| K | G or T |
| M | A or C |
| B | C or G or T |
| D | A or G or T |
| H | A or C or T |
| V | A or C or G |
| N | any nucleotide (base) |
| . or - | interval |

**Table 3**

| Codice IUPAC | Amino acid |
|---|---|
| A | Alanine |
| C | Cysteine |
| D | Aspartic Acid |
| E | Glutamic Acid |
| F | Phenylalanine |
| G | Glycine |
| H | Histidine |
| I | Isoleucine |
| K | Lysine |
| L | Leucine |
| M | Methionine |
| N | Asparagine |
| P | Proline |
| Q | Glutamine |
| R | Arginine |
| S | Serine |
| T | Threonine |
| V | Valine |
| W | Tryptophan |
| Y | Tyrosine |

Nucleotide sequences SEQ.ID.NO.1 through SEQ.ID.NO.40 shown in Annex 1 for the *imp* gene of the strains of Table 1 can be represented by means of a single nucleotide sequence (so called "consensus" sequence) indicated as SEQ.ID.NO.81 in Annex 3 e, similarly, the corresponding amino acid sequences SEQ.ID.NO.41 through SEQ.ID.NO.80 of the IMP protein shown in Annex 2 can be represented by means of a single ammino acid "consensus" sequence indicated as SEQ.ID.NO.82 in Annex 4.

As is known, "consensus" sequence means a sort of "average" sequence (of nucleotides or amino acids) resulting from the comparison of many similar and functionally equivalent sequences. In the specific instance, consensus sequences have been obtained by using the program "CLC Sequence Viewer", of the company CLCbio/Qiagen.

Also the sequences SEQ.ID.NO.81 and SEQ.ID.NO.82 are expressed according to the IUPAC nomenclature. Therefore, for indicating the nucleotide consensus sequence the abbreviations shown in Table 2 have been used for indicating polymorphism (i.e. the presence of different nucleotides in the same position in different sequences). With respect to the amino acid consensus sequence, the IUPAC nomenclature does not provide for any way of indicating the "coexistence" of different amino acids in different sequences. The consensus sequence has therefore been generated by choosing the "majority" option, i.e. if there are different amino acids in the same position in the sequences SEQ.ID.NO.41 - SEQ.ID.NO.80, the most present amino acid has been indicated.

Further disclosed are primers that can be used for characterizing the *imp* gene of phytoplasmas of the group 16SrV. In particular, two forward primers are provided, indicated as DnaDFDfl and DnaDFDf2 in Fig. 1, and two revers primers are provided, indicated as PyrGFDr1 and PyrGFDr2 in Fig. 1, whose nucleotide sequences are shown as SEQ.ID.NO.83, 84, 85 and 86 in Annex 7.

In Fig. 1 it is visible that the two pairs of primers are shown at the *dnaD* and *pyrG* genes, in such a position that the two primers DnaDFDfl and PyrGFDr1 (indicated by a solid line) allow to amplify, when used in a PCR reaction, a zone having a length of about 1000 base pairs, and the two primers DnaDFDf2 and PyrGFDr2 (indicated by a dashed line) allow to amplify a zone having a length of about 800 base pairs.

The primers DnaDFDfl and DnaDFDf2 are fragments of nucleotide sequences of the *dnaD* gene of some strains of Table 1 (as evidenced in Annex 1 by the sequence portions located inside a frame and underlined with simple underline or double underline, respectively), whereas the primers PyrGFDr1 and PyrGFDr2 are fragments complementary to the portions of the sequences of Annex 1 that are indicated by a thin and thick wavy underline, respectively, and are similarly located inside a frame.

Once the primers are given, the sequences SEQ.ID.NO.1 - SEQ.ID.NO.40 of the strains of Table 1 and, in general, of all the phytoplasmas of the sub-groups 16SrV-C, 16SrV-D and 16SrV-E, as well as the nucleotide consensus sequence SEQ.ID.NO.81 can be obtained univocally and without any inventive effort by any person skilled in the art.

The choice of drawing the primers at the genes flanking the *imp* gene, as is clear to the person skilled in the art, is due primarily to the low polymorphism of the *dnaD* and *pyrG* genes, which are much more similar in different organisms (Kakizawa et al., 2009, [17]), whereby it is easy to work on these flanking genes in order to obtain effective primers functioning for all the strains in a PCR reaction. On the contrary, it would have been almost impossible to design on the *imp* gene efficient primers functioning on other strains, because, as mentioned, said gene is one of the most polymorphic genes in phytoplasmas.

A second and essential advantage lies in the fact that, with the indicated position, the genes surely amplify the whole *imp* gene of interest for the present invention and they provide information about the whole gene. If the primers were designed directly on the gene of interest, as is usual with genes having limited polymorphism, through the PCR reaction it would be impossible to obtain the whole imp gene and, consequently, the information about the whole IMP protein that are necessary for developing an efficient enzymatic assay; without the whole protein it is much more difficult to develop an efficient enzyme assay.

The experimental procedure by which the primers SEQ.ID.NO.83 - SEQ.ID.NO.86 have been obtained is briefly described below:
1) the *dnaD-imp-pyrG* gene portion already sequenced in other phytoplasmas has been sought;
2) the sequences found have been aligned and the most conserved portions of the *dnaD* and *pyrG* have been sought;
3) a series of primers (about twenty, 8-12 per gene) has been provided on these portions;
4) all the primers have been tested in parallel on some strains of phytoplasmas, while changing the PCR conditions, until one or more "amplification bands", i.e. fragments of the gene of the phytoplasma of interest, have been obtained;
5) some of the obtained fragments of the gene of the phytoplasma (in particular those obtained from FD70, which is the longest sequence just because of this) have been isolated and sequenced;
6) on this new sequence obtained, which is a sequence of a FD strain, new primers have been designed on the *dnaD* and *pyrG* genes, which are more similar to the sequence of the other FD strains;
7) the new primers have been tested on many strains, also of different phytoplasmas, in order to check that they diagnosticate all the strains of interest and do not diagnosticate other phytoplasmas;
8) the best primers have been chosen (in the present instance, two pairs), which have been mutually "combined" in order to give even better results. For this reason, in order to obtain a more efficient reaction, the two outermost primers (DnaDFDfl and PyrGFDr1) and subsequently the two innermost primers (DnaDFDf2 and PyrGFDr2) have been used in a nested PCR reaction;
9) the best primers have been used for obtaining all the other sequences of interest.

As mentioned above, the invention further relates to the use of the gene sequence, the amino acid sequence for developing diagnostic methods for recognizing pathologies caused by phytoplasmas of the group 16SrV and, in particular, for recognizing flavescence dorée of grapevine.

In a first instance, at least two of the primers mentioned above can be used in a known manner in a PCR reaction in order to amplify the nucleotide regions of the *imp* gene of the phytoplasmas of the sub-groups 16SrV-C, 16SrV-D and 16SrV-E. As is known, PCR is a molecular assay based on the chain reaction of an enzyme, polymerase, which, in the presence of two initiators or primers, free deoxynucleotides (dNTP), and MgCl2, and under specific thermal conditions and with a suitable reaction buffer, adheres to the complementary DNA regions of a sample and synthetize the DNA filament comprised between the two primers. The PCR reaction consists of three steps: denaturation of the DNA double helix; annealing, i.e. attack by the reagents against the DNA filament; synthesis and extension of the filaments. These three steps are repeated cyclically several times (30-40), until a large amount of the DNA fragment comprised between the two primers is obtained as reaction product. This specific product can be visualized in gel (agarose, acrylamide, etc.) through specific coloring (ethidium bromide, other DNA intercalators). If the product is amplified and visualized, the sample is positive, and therefore in the specific instance it contains the pathogen containing the DNA sequence SEQ.ID.NO.41. If the PCR product is not amplified, this means that the sample is negative.

In particular, by using the pair of primers DnaDFDf1/PyrGFDr1 (SEQ.ID.NO.83, SEQ.ID.NO.85) a genomic region of about 1000 base pairs can be amplified which includes the terminal part of the *dnaD* gene, the entire *imp* gene, the terminal part of the *pyrG* gene and the two intergenic sequences comprised therebetween. If, instead, the pair of primers DnaDFDf2/PyrGFDr2 (SEQ.ID.NO.84, SEQ.ID.NO.86) is used, a genomic region of about 800 base pairs is amplified which comprises a smaller terminal part of the *dnaD* gene, the entire imp gene, a smaller terminal part of the *pyrG* gene and the two intergenic sequences comprised therebetween. Both pairs of primers can be used in two subsequent different assays, i.e. in a nested PCR reaction. Another possibility consists in using the primer DnaDFDfl in combination with the primer PyrGFDr2, or the prime DnaDFDf2 in combination with the primer PyrGFDr1: in this case different nucleotide portions of the *dnaD* and *pyrG* genes, the entire *imp* gene and the intergenic portions are amplified.

The PCR reaction and its use are well known to persons skilled in the art and further details are superfluous.

Of course, the primers can be used not only in PCR assays, but also in RT-PCR (Reverse Transcriptase PCR) assays, real-time PCR, as molecular probes in hybridization assays, in microarrays or other molecular assays.

Another possible use of the PCR product, as an alternative to visualization, is sequencing it by means of a suitable equipment (DNA sequencers), in order to obtain the exact sequence of the fragment. In this way identification of the sequence specific for the *imp* gene (and for the surrounding gene regions) of the invention is obtained.

On the basis of the nucleotide sequence SEQ.ID.NO.41 (and therefore of any of the sequences SEQ.ID.NO.1 through SEQ.ID.NO.40), the corresponding proteins can be synthetized *in vitro* by inserting the sequences of interest into a suitable plasmid which is then inserted into a bacterium such as *Escherichia coli.* In this way, IMP proteins present in the membrane of the phytoplasmas of the group 16SrV can be obtained synthetically. From them it is possible to obtain, by means of various techniques well described in literature, monoclonal or polyclonal antibodies that are then used for developing serological diagnostic kits, for instance ELISA assays.

The ELISA assay is a sensitive technique in which binding of an antigen to its specific antibody is made visible by using an enzyme covalently bound to the antibody and capable of catalyzing a reaction which transforms the substrate into a colored product. The are two basic ELISA methods: direct and indirect. In the direct method, the individuation of the presence of the target protein, in the present instance of the pathogen, takes place by means of addition of primary antibodies, called conjugated antibodies, marked with the enzyme and capable of binding to the viral antigen in wells containing infected samples. In the indirect method, two kinds of conjugated antibodies are used: primary antibodies specific for the virus to be searched; secondary anti-immunoglobuline antibodies, marked with the enzyme and capable of binding to primary antibodies remaining where the antigen is. Several variants of the method have been introduced that makes it flexible for use and adaptable to the individuation of pathogens in the different environments where they are present.

For instance, in the method DAS-ELISA (DAS = Double-Antibody Sandwich,) a first specific antibody is adsorbed to the walls of the wells of a polystyrene plate (coating); the samples to be assayed and the conjugated antibodies are added afterwards. The antigene revelation can take place either directly or indirectly, depending on whether the primary antibodies are marked or secondary conjugated antibodies are added (TAS-ELISA, Triple-Antibody Sandwich). In the method PTA-ELISA (PTA = Plate-Trapped Antigen) the antigen is made adsorbed directly on the surface of the plate wells and is then recognized by adding a specific primary antibody, followed by a secondary antibody conjugated with an enzyme and by the corresponding substrate. An ELISA assay consists of six steps: sensitization (not required for PTA-ELISA), preparation of samples, distribution of samples, conjugation (simple or double), addition of the substrate, reading of the results.

There are other serological assays that are based on amino acid sequences and involve antibodies, mainly derived from the more common ELISA assay.

These assays, too, are well known to persons skilled in the art and further details are superfluous.

It is evident that the above description has been given by way of non-limiting example. In particular, the nucleotide sequences reported herein also allow to design primers different from those of the sequences SEQ.ID.NO.83 - SEQ.ID.NO.86, which, however, have the same function and are still designed at the *dnaD* and *pyrG* genes and can therefore be used in the same ways as the described ones.

### BIBLIOGRAPHY

[1] Angelini E., Clair D., Borgo M., Bertaccini A., Boudon-Padieu E., 2001. Flavescence doree in France and Italy - Occurrence of closely related phytoplasma isolates and their near relationships to Palatinate grapevine yellows and an alder phytoplasma. Vitis 40, 79-86.
[2] Angelini E., Squizzato F., Lucchetta G., Borgo M., 2004. Detection of a phytoplasma associated with grapevine Flavescence dorée in Clematis vitalba. Eur. J. Plant Pathol, 110: 193-201.
[3] Angelini E., 2010. Field assessment and diagnostic methods for detection of grapevine phytoplasmas. In (Del Rot S. ed.): Methodologies and results in grapevine research, Springer Verlag, 247-258, ISBN 978-90-481-9282-3.
[4] Arnaud G., Malembic-Maher S., Salar P., Bonnet P., Maixner M., Marcone C., Boudon-Padieu E., Foissac X., 2007. Multilocus sequence typing confirms the close genetic interrelate,, mjmmn nnedness of three distinct Flavescence dorée phytoplasma strain clusters and group 16SrV phytoplasmas infecting grapevine and alder in Europe. Applied and Environmental Microbiology 73, 4001-10.
[5] Batlle, A., Laviña, A., Clair, D., Larrue, J., Kuszala, C., Boudon-Padieu, E., 1997. Detection of Flavescence dorée in grapevine in Northern Spain. Vitis 36 (4), 211-212.
[6] Belli G., Fortusini A., Osler R., Amici A., 1973. Presenza di una malattia del tipo Flavescence dorée in vigneti dell'Oltrepò Pavese. Rivista di Patologia Vegetale 9, 51-56.
[7] Botti S., Bertaccini A 2007. Grapevine yellows in Northern Italy: molecular identification of Flavescence dorée phytoplasma strains and of Bois Noir phytoplasmas. Journal of Applied Microbiology 103, 2325-30.bh
[8] Boudon-Padieu E., Larrue J., Caudwell A., 1989. ELISA and Dot-Blot detection of Flavescence dorée MLO in individual leafhopper vectors during latency and inoculative state. Curr. Microbiol. 19, 357-364.
[9] Caudwell A., 1957. Deux années d'études sur la Flavescence dorée, nouvelle maladie grave de la vigne. Annales de L' Amélioration des Plantes 4: 359-363.
[10] Contaldo N., Bertaccini A., Paltrinieri S., Windsor HM, Windsor G.D., 2012. Axenic culture of plant pathogenic phytoplasmas Phytopathologia Mediterranea 51, 607-617.
[11] De Sousa E., Casati, P. Cardoso F., Baltazar C., Durante G., Quaglio F., Bianco P.A., 2009. Flavescence dorée phytoplasma affecting grapevine (Vitis vinifera) newly reported in Portugal. New Disease Reports 19, 33.
[12] Duduk B., Botti S., Ivanovic M., Dukic N., Bertaccini A., 2003. Molecular characterization of a Flavescence dorée phytoplasma infecting grapevine in Serbia. Proceedings of the XIV Meeting of the International Council for the Study of Virus and Virus-like Diseases of the Grapevine, 91-92.
[13] Filippin L., Jovič J., Cvrkovič T., Forte V., Clair D., Toš̌̌evski I., Boudon-Padieu E., Borgo M., Angelini E., 2009. Molecular characteristics of phytoplasmas associated with Flavescence dorée in clematis and grapevine and preliminary results on the role of Dictyophara europaea as a vector. Plant Pathol., 58: 826-837.
[14] Filippin L., Borgo M., Angelini E., 2010. Identification of FD phytoplasma in plants of Ailanthus altissima in Italy. Book of Abstracts COST FA0807 Meeting "Current status and perspectives of phytoplasma disease research and management" Sitges (Spain), 1-2 February 2010, 14.
[15] Gugerli P., Besse S., Colombi L., Ramel M.E., Rigotti S., Cazelles O., 2006. First out break of Flavescence dorée (FD) in Swiss vineyards. Proceedings of the 15th Meeting of the International Council for the Study of Virus and Virus-like Disease of the Grapevine (ICVG) addendum. IRPCM, 2004. "Candidatus Phytoplasma", a taxon for the wall-less, non-helical prokaryotes that colonize plant phloem and insects. International Journal of Systematic and Evolutionary Microbiology 54, 1243-55.
[16] Kakizawa S. Oshima K., Namba S., 2006. Diversity and functional importance of phytoplasma membrane proteins. Trends in Microbiology 14, 254-256.
[17] Kakizawa S., Oshima K., Ishii Y., Hoshi A., Maejima K., Jung H.-Y., Yamaji Y., Namba S., 2009. Cloning of immunodominant membrane protein genes of phytoplasmas and their in planta expression. FEMS Microbiology Letters 293, 92-101.
[18] Kuzmanovič S., Martini M., Ermacora P., Ferrini F., Starovič M., Tosič M., Carraro L., Osler R., 2008. Incidence and molecular characterization of Flavescence dorée and stolbur phytoplasmas in grapevine cultivars from different viticultural areas of Serbia. Vitis 47, 105-11.
[19] Lee I.M., Gundersen-Rindal D.E., Davis R.E., Bartoszyk I.M., 1998. Revised classification scheme of Phytoplasmas based on RFLP analyses of 16S rRNA and ribosomal protein gene sequences. Int. J. Syst. Bacteriol. 48, 1153-1169.
[20] Maixner M., Reinert W., 1999. Oncopsis alni (Schrank) (Auchenorrhyncha: Cicadellidae) as a vector of the alder yellows phytoplasma of Alnus glutinosa (L.) Gaertn. European Journal of Plant Pathology 105, 87-94.
[21] Maixner M., 2006. Grapevine yellows: current developments and unsolved questions.; Proceedings of the 15th Meeting of the International Council for the Study of Virus and Virus-like Disease of the Grapevine (ICVG) addendum.
[22] Martini M., Botti S., Marcone C., Marzachì C., Casati P., Bianco P.A., Benedetti R., Bertaccini A., 2002. Genetic variability among Flavescence dorée phytoplasmas from different origins in Italy and France. Molecular and Cellular Probes 16, 197-208.
[23] Mehle N., Seljak G., Rupar M., Ravnikar M., Dermastia M., 2010. The first detection of a phytoplasma from the 16SrV (Elm yellows) group in the mosaic leafhopper Orientus ishidae. New Disease Reports 22, 11.
[24] Mori N., Bressan A., Martini M., Guadagnini M., Girolami V., Bertaccini A., 2002. Experimental transmission by Scaphoideus titanus Ball of two Flavescence dorée-type phytoplasmas. Vitis 41, 99-102.
[25] Reisenzein H., Steffek R. 2011. First outbreaks of grapevine 'flavescence dorée' in Austrian viticulture. Bulletin of Insectology 64 (Supplement): S223-S224.
[26] Schvester D., Carle P., Moutous G., 1961. Sur la transmission de la flavescence dorée des vignes par une cicadelle. Comptes Rendus des Séances de l'Académie d'Agriculture de France 47, 1021-4.
[27] Seddas A., Meignoz R., Daire X., Boudon-Padieu E., 1996. Generation and characterization of monoclonal antibodies to Flavescence dorée phytoplasma: serological relationships and differences in electroblot immunoassay profiles of Flavescence dorée and elm yellows phytoplasmas. Eur J Plant Pathol 102:757-764.
[28] Seemüller E., Marcone C., Lauer U., Ragozzino A., Göschl M., 1998. Current status of molecular classification of the phytoplasmas. J. Plant Pathol. 80 (1), 3-26.
[29] Š̌eruga Music M., Škoric D., Haluska I., Krizanac I., Plavec J., Mikec I., 2011. First report of 'flavescence dorée' - related phytoplasma affecting grapevines in Croatia. Plant Disease, 95: 353.
[30] Sambrok J. and Russel D.W., 2001. Chapter 8. In vitro amplification of DNA by the polymerase chain reaction. In: "Molecular cloning, a laboratory manual", third edition, Cold Spring Harbor Laboratory Press, Vol. 2: 8.13-8.14.
[31] Mitsuhashi M., 1996. Technical report: Part 2. Basic requirements for designing optimal PCR Primers. J. Clin. Lab. Anal. 10: 258-293.

### ANNEX 1

### Alignment of nucleotide sequence of the dnaD-imp-pyrG gene portion of strains of phytoplasmas belonging to the ribosomal group 16SrV

### ANNEX 2

### Alignment of amino acid sequences of the IMP protein of strains of phytoplasmas belonging to the ribosomal 16SrV

### ANNEX 3

Nucleotide consensus sequence of the *imp* gene of strains of phytoplasmas belonging to the ribosomal group 16SrV (SEQ.ID.NO.81)

### ANNEX 4

Amino acid consensus sequence of the IMP protein of strains of phytoplasmas belonging to the ribosomal group 16SrV (SEQ.ID.NO.82)

### ANNEX 5

Partial sequences belonging to the *dnaD* and *pyrG* genes and used as primers in an aspect of this disclosure
SEQ.ID.NO.83 (DnaDFDf1): 5'-TAG AGA GAA TTT TAG GCC ACG-3'
SEQ.ID.NO.84 (DnaDFDf2): 5'-ATA GAA AAT AAC GAT AAA GCA G-3'
SEQ.ID.NO.85 (PyrGFDr1): 5'-AAT AAT GAA GAA CAA TTA CCT G-3'
SEQ.ID.NO.86 (PyrGFDr2): 5'-TCA AGA CCT TTT AAA CCA CAC CC-3'

## Claims

1. Nucleotide sequence according to SEQ.ID.NO.81, or the sequence complementary to SEQ.ID.NO.81, coding for the *imp* gene of phytoplasmas belonging to the ribosomal group 16SrV.

2. Nucleotide sequence comprising:
- the nucleotides of a sequence according to SEQ.ID.NO.81, or of the sequence complementary to SEQ.ID.NO.81, coding for the *imp* gene of phytoplasmas belonging to the ribosomal group 16SrV;
- a nucleotide sequence, or the complementary sequence, belonging to a terminal part of the *dnaD* gene preceding the *imp* gene and coding for a protein involved in the start of chromosomal replication;
- a nucleotide sequence, or the complementary sequence, belonging to a terminal part of the *pyrG* gene following the *imp* gene and coding for cytidine triphosphate synthetase; and
- the intergenic sequences between the *dnaD* and *imp* genes and between the *imp* and *pyrG* genes.

3. Nucleotide sequence according to Claim 2, which is one of the sequences SEQ.ID.NO.1 through SEQ.ID.NO.40 or the sequence complementary to said one of the sequences SEQ.ID.NO.1 through SEQ.ID.NO.40, respectively.

4. Amino acid sequence according to SEQ.ID.NO.82, coding for immunodominant membrane proteins, IMP proteins, of phytoplasmas belonging to the ribosomal group 16SrV.

5. Amino acid sequence according to Claim 4, which is one of the sequences SEQ.ID.NO.41 through SEQ.ID.NO.80.

6. Use of the nucleotide sequences or of the complementary sequences according to any of the Claims 1 to 3, or of the amino acid sequences according to any of Claims 4 or 5 for the diagnosis of plant diseases induced by phytoplasmas belonging to the ribosomal group 16SrV.

7. Use according to Claim 6 for the diagnosis of flavescence dorée of grapevine.

## Patentansprüche

1. Nukleotidsequenz gemäß SEQ.ID.NO.81 oder der zu SEQ.ID.NO.81 komplementären Sequenz, die für das *imp* Gen von Phytoplasmen kodiert, die zu der ribosomalen Gruppe 16SrV gehören.

2. Nukleotidsequenz mit:
- den Nukleotiden einer Sequenz gemäß SEQ.ID.NO.81 oder der zu SEQ.ID.NO.81 komplementären Sequenz, die für das *imp* Gen von Phytoplasmen kodiert, die zu der ribosomalen Gruppe 16SrV gehören,
- einer Nukleotidsequenz oder der komplementären Sequenz, die zu einem Endabschnitt des *dnaD* Gens gehört, das dem *imp* Gen vorangeht und für ein Protein kodiert, das mit dem Beginn der chromosomalen Replikation zu tun hat,
- einer Nukleotidsequenz oder der komplementären Sequenz, die zu einem Endabschnitt des *pyrG* Gens gehört, das auf das *imp* Gen folgt und für Cytidintriphosphat-Synthetase kodiert, und
- die intergenischen Sequenzen zwischen dem *dnaD* Gen und *imp* Gen sowie zwischen dem *imp* Gen und dem *pyrG* Gen.

3. Nukleotidsequenz nach Anspruch 2, die eine der Sequenzen SEQ.ID.NO.1 bis SEQ.ID.NO.40 oder der komplementären Sequenzen zu einer der Sequenzen SEQ.ID.NO.1 bis SEQ.ID.NO.40 ist.

4. Aminosäuresequenz gemäß SEQ.ID.NO.82, die für immunodominante Membranproteine, IMP Proteine, von Phytoplasmen kodiert, die zu der ribosomalen Gruppe 16SrV gehören.

5. Aminosäuresequenz nach Anspruch 4, die eine der Sequenzen SEQ.ID.NO.41 bis SEQ.ID.NO.80 ist.

6. Verwendung der Nukleotidsequenzen oder der komplementären Sequenzen nach einem der Ansprüche 1 bis 3 oder der Aminosäuresequenzen nach einem der Ansprüche 4 oder 5 zur Diagnose von Pflanzenkrankheiten, die durch Phytoplasmen ausgelöst werden, die zu der ribosomalen Gruppe 16SrV gehören.

7. Verwendung nach Anspruch 6 zur Diagnose von goldgelber Vergilbung von Weinreben.

## Revendications

1. Séquences de nucléotides selon SEQ ID N° 81, ou séquence complémentaire de SEQ ID N° 81, codant pour le gène *imp* de phytoplasmes appartenant au groupe ribosomique 16SrV.

2. Séquences de nucléotides comprenant :
- les nucléotides d'une séquence selon SEQ ID N° 81, ou de la séquence complémentaire de SEQ ID N° 81, codant pour le gène *imp* de phytoplasmes appartenant au groupe ribosomique 16SrV ;
- une séquence de nucléotides, ou la séquence complémentaire, appartenant à une partie terminale du gène *dnaD* précédant le gène *imp* et codant pour une protéine impliquée dans le départ d'une réplication chromosomique ;
- une séquence de nucléotides, ou la séquence complémentaire, appartenant à une partie terminale du gène *pyrG* suivant le gène *imp* et codant pour la cytidine triphosphate synthétase ; et
- les séquences intergéniques entre les gènes *dnaD* et *imp* et entre les gènes *imp* et *pyrG.*

3. Séquences de nucléotides selon la revendication 2, qui est l'une des séquences SEQ ID N° 1 à SEQ ID N° 40 ou la séquence complémentaire de ladite une des séquences SEQ ID N° 1 à SEQ ID N° 40, respectivement.

4. Séquence d'acides aminés selon SEQ ID N° 82, codant pour des protéines membranaires immunodominantes, protéines IMP, de phytoplasmes appartenant au groupe ribosomique 16SrV.

5. Séquence d'acides aminés selon la revendication 4, qui est l'une des séquences SEQ ID N° 41 à SEQ ID N° 80.

6. Utilisation des séquences de nucléotides ou des séquences complémentaires selon l'une quelconque des revendications 1 à 3, ou des séquences d'acides aminés selon l'une quelconque des revendications 4 ou 5, pour le diagnostic de maladies de plantes induites par des phytoplasmes appartenant au groupe ribosomique 16SrV.

7. Utilisation selon la revendication 6, pour le diagnostic de la flavescence dorée de la vigne.
